(19) Europäisches Patentamt
European Patent Office
Office européen des brevets

(11) Numéro de publication : **0 360 820 B1**

# (12) FASCICULE DE BREVET EUROPEEN

(45) Date de publication du fascicule du brevet : **19.08.92 Bulletin 92/34**

(21) Numéro de dépôt : **88904231.3**

(22) Date de dépôt : **29.04.88**

(86) Numéro de dépôt international : **PCT/FR88/00209**

(87) Numéro de publication internationale : **WO 88/08419 03.11.88 Gazette 88/24**

(51) Int. Cl.$^5$ : **C07D 213/22,** C07D 213/30, C07D 213/79, C07H 19/067, C07H 19/167, B01J 19/00

## (54) LIGANDS D'OLIGOBIPYRIDINES, PROCEDES D'OBTENTION ET APPLICATION A TITRE D'AGENTS COMPLEXANTS.

(30) Priorité : **30.04.87 FR 8706202**

(43) Date de publication de la demande : **04.04.90 Bulletin 90/14**

(45) Mention de la délivrance du brevet : **19.08.92 Bulletin 92/34**

(84) Etats contractants désignés : **AT BE CH DE FR GB IT LI LU NL SE**

(56) Documents cités :
**Proceedings of the National Academy of Sciences, vol. 84, no. 9, mai 1987; J.-M. LEHN et al.: "Spontaneous assembly ofdouble-stranded helicates from oligobipyridine ligands and copper (I) cations: structure of an inorganic double helix", pp.2565-2569**
**Journal of Medicinal Chemistry, vol. 18, no. 11, novembre 1975; D.B. TAYLOR et al.: "Synthesis of a bifunctional coordinationcomplex of osmium with curariform activity", pp. 1088-1094**
**G.R. NEWKOME: "Pyridine and its derivatives", partie 5, 1984, J. Wiley & Sons, New York, USA, pp. 524-525**
**Accounts of Chemical Research, vol. 19, juin 1986, American Chemical Society, USA; D.S. SIGMAN: "Nuclease activity of1,10-phenanthroline-copper ion", pp. 180-186**

(73) Titulaire : **CIS BIO INTERNATIONAL RN 306 F-91000 Saclay (FR)**

(72) Inventeur : **LEHN, Jean-Marie 21, rue d'Oslo F-67000 Strasbourg (FR)**
Inventeur : **RIGAULT, Annie 1, rue de Neufchatel F-67000 Strasbourg (FR)**
Inventeur : **SIEGEL, Jay Chemistry Department University of California San Diego La Jolla, CA 92093 (US)**
Inventeur : **HARDING, Margaret 11, rue J.J.-Henner F-67000 Strasbourg (FR)**
Inventeur : **HE, Yong, Bing 11, rue Louvois F-67000 Strasbourg (FR)**

(74) Mandataire : **Gillard, Marie-Louise et al Cabinet Beau de Loménie 55, Rue d'Amsterdam F-75008 Paris (FR)**

EP 0 360 820 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une nouvelle classe de ligands organiques, à savoir les ligands d'oligobipyridines, qui sont d'excellents agents complexants d'ions métalliques. On notera que la plupart de ces ligands, par liaison avec des ions métalliques ayant une géométrie de coordination donnée, se transforment spontanément en des complexes polymétalliques ayant la structure d'une hélice à double brin, similaire à la structure des acides nucléiques.

Des travaux antérieurs ont montré que certains complexes métalliques avec des ligands à base de pyridine, tels que par exemple le complexe de formule $[Cu_2(pQP)_2](ClO_4)_2$, dans laquelle pQP désigne la quaterpyridine perpendiculaire, présentaient une géométrie de coordination tétraédrique déformée. A cet effet, on peut se reporter aux articles de J.M. LEHN et al. dans "Nouveau Journal de Chimie" vol. 7 n° 7 (1983) p 413-420 et de G.C. VAN STEIN et al. dans "Inorganic Chemistry", vol. 23 n° 25 (1984) p 4269-4278.

On a maintenant trouvé des nouveaux ligands à base de bipyridine, qui sont capables de complexer les ions métalliques.

Ces nouveaux ligands sont d'excellents agents complexants d'ions métalliques qui trouvent une application dans tous les domaines où il est souhaitable de complexer ces ions par exemple en vue de leur extraction, séparation, concentration ou valorisation.

De plus, étant donné la structure géométrique particulière adoptée par certains ligands de l'invention, ceux-ci peuvent être utilisés dans le domaine biologique par exemple pour couper l'ADN selon des caractéristiques particulières et comme agents intercalants.

Les ligands d'oligobipyridines selon l'invention répondent à la formule générale :

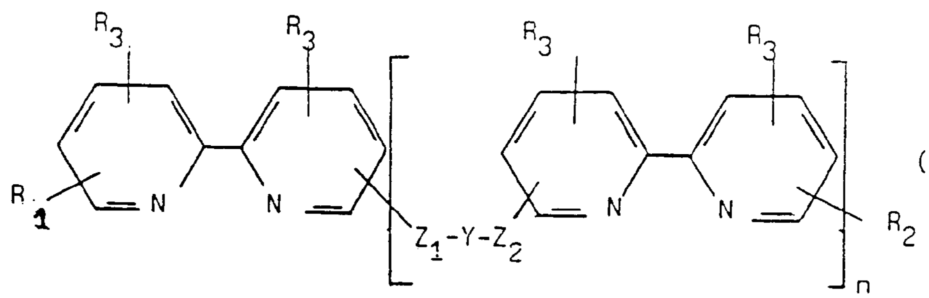

(I)

dans laquelle :

- $Z_1$ et $Z_2$, identiques ou différents, représentent des groupes alkylène en $C_1$-$C_{12}$ ;
- Y est l'oxygène ou le soufre ; ou bien
- $Z_1$-Y-$Z_2$- représente les groupes $-CH = CH-$ ou $-CH_2 - CH_2-$
- n est un nombre entier compris entre 1 et 6 ;
- $R_1$ et $R_2$, identiques ou différents, représentent des groupes alkyle en $C_1$ - $C_5$ en particulier le groupe méthyle ou le groupe éthyle, les radicaux $-Z_1YH$, $-Z_2YH$, $-Z_1X$ ou $-Z_2X$ dans lesquels Y est tel que défini ci- dessus et X est un atome d'halogène ou un groupe de formule

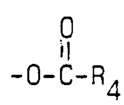

dans laquelle $R_4$ est un groupe alkyle en $C_1$ - $C_5$.

- $R_3$ est l'hydrogène ou un groupe $-COR_5$ dans lequel $R_5$ représente :
  . un halogène, de préférence le chlore ;
  . un groupe $OR_5$ où $R_5$ représente l'hydrogène, ou un groupe alkyle en $C_1$ - $C_5$ ; ou
  . un groupe $NR_7R_8$ dans lequel $R_7$ et $R_8$, identiques ou différents, représentent l'hydrogène ou un groupe alkyle en $C_1$ - $C_5$ ou bien l'un d'eux représente l'hydrogène et l'autre est un nucléoside.

Par "nucléoside" on désigne dans la présente description les composés adénosine, cytidine, thymidine, guanosine et uridine.

Les groupes bipyridines des ligands selon l'invention sont, de préférence substitués en position 4,4′ par les groupes $R_3$ et en position 5,5′ ou 6,6′ par l'ensemble des groupes $R_1$, $R_2$,$Z_1$, $Z_2$.

Les procédés pour l'obtention des ligands selon l'invention sont illustrés par les schémas réactionnels ci-

après :

Schéma A (cas où n = 1, Y = O ; $Z_1 = Z_2$ = alkylène)

M = métal

X = halogène

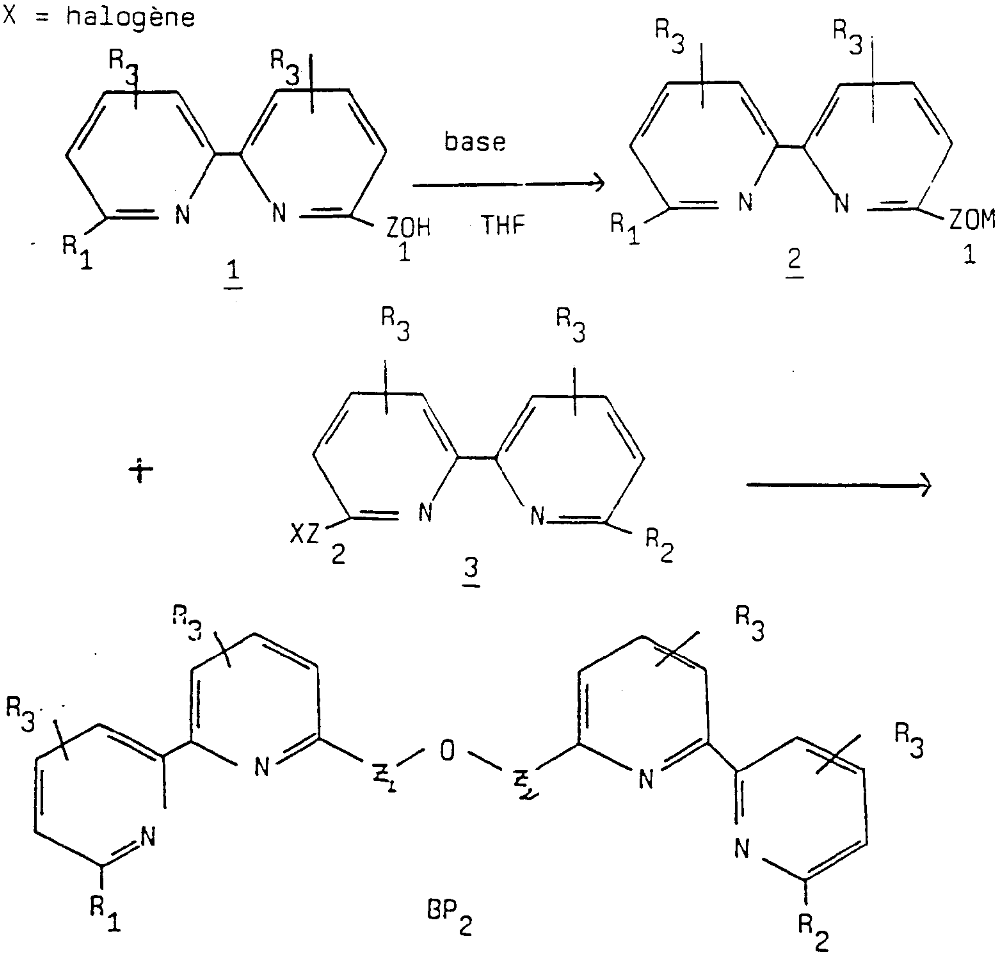


1 2 3 $BP_2$

Ce procédé consiste tout d'abord à transformer le composé hydroxylé 1 en un sel métallique 2 par action d'une base, tel qu'un hydrure métallique ou d'un composé organométallique en solution dans un solvant approprié, tel que le tétrahydrofuranne (THF). On peut avantageusement utiliser l'hydrure de sodium, le butyllithium ou le tertiobutylate de potassium.

Dans le cas où l'on utilise un hydrure métallique, la réaction est effectuée sous atmosphère inerte à la température ambiante.

Lorsqu'on utilise un composé organométallique, on opère également sous atmosphère inerte à très basse température, le composé organométallique étant mis en oeuvre dans un solvant approprié, par exemple l'hexane.

Ensuite, on fait réagir au reflux pendant 10 à 15 heures le sel métallique 2 avec un équivalent du dérivé monohalogéné 3. On obtient alors l'oligobipyridine $BP_2$ que l'on isole du milieu réactionnel par des moyens classiques de séparation.

Schéma B (cas où n = 2 ; Y = O ; $Z_1 = Z_2$ = alkylène

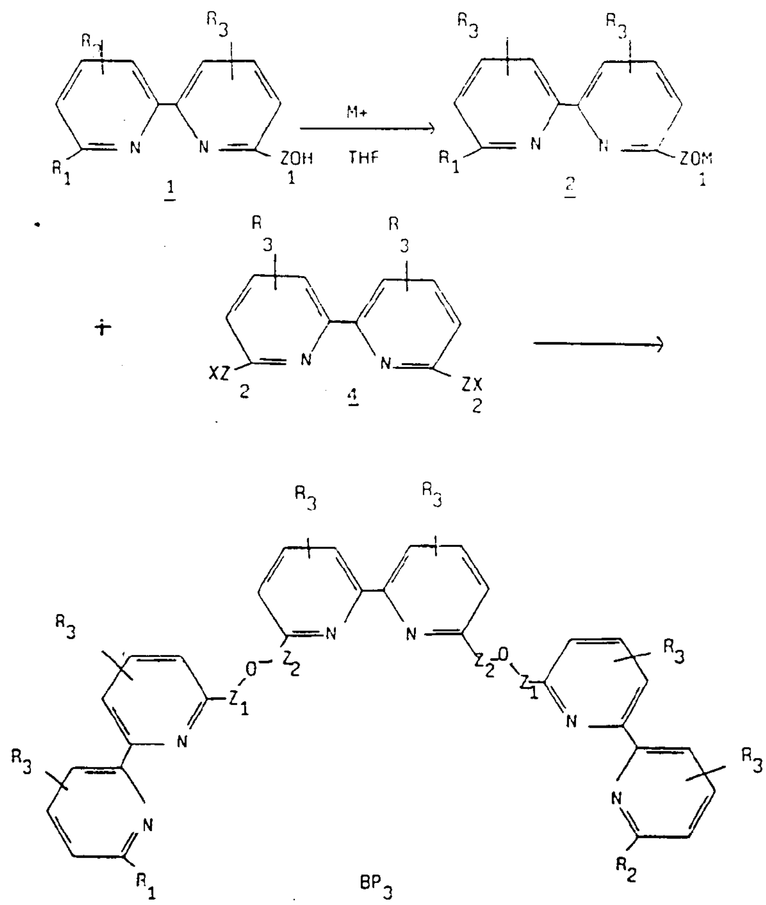


BP$_3$

La première étape de ce procédé est identique à la première étape du schéma réactionnel A. La seconde étape consiste à faire réagir le sel métallique 2 avec le dérivé dihalogéné 4. Cette réaction est réalisée au reflux pendant 15 à 20 heures. L'oligobipyridine $BP_3$ est ensuite isolée du milieu réactionnel par des moyens classiques de séparation.

Pour l'obtention des oligobipyridines supérieures (n = 3 à 6), on procède selon l'un des schémas réactionnels ci-dessus en préparant d'abord le dérivé monohydroxylé de l'oligobipyridine $BP_2$ ou $BP_3$ lequel est transformé en le sel métallique correspondant qui est mis ensuite à réagir avec soit le dérivé monohalogéné 3 ou le dimère ou trimère correspondant monohalogéné, soit le dérivé dihalogéné 4.

Les procédés pour l'obtention des composés ce l'invention de formule I dans laquelle $Z_1$ et $Z_2$ sont un groupe alkylène ont été décrits en détail ci-dessus pour les composés de formule I où Y est l'oxygène. Les composés de formule I où Y est le soufre peuvent être obtenus selon des procédés analogues à la portée de l'homme de l'art.

Schéma C (Cas où n = 2 ; - $Z_1$-Y-$Z_2$- est -CH=CH-)

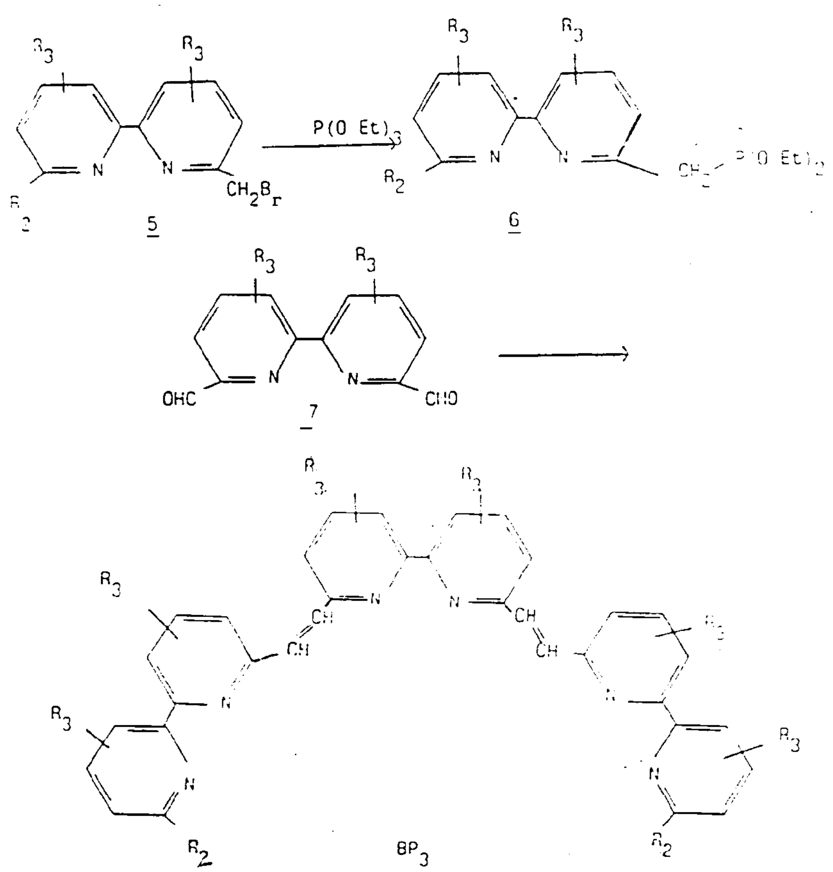


5 6 7 $BP_3$

Ce procédé consiste tout d'abord à transformer le dérivé bromométhyle 5 en le phosphonate 6 par action du triéthylphosphite à température de l'ordre de 150° C et sous atmosphère inerte, puis à coupler le phosphonate ainsi obtenu avec le dialdéhyde 7. Cette réaction est réalisée au sein d'un solvant anhydre approprié, tel que le THF en présence d'un hydrure métallique, tel que l'hydrure de sodium. On opère avantageusement au reflux.

Les composés de départ 5 et 7 peuvent être préparés par des procédés classiques bien connus de l'homme de l'art et notamment par les procédés décrits dans : J Organometallic Chemistry 56 (1973) 53-66.

L'oligobipyridine de formule I dans laquelle -$Z_1$-Y-$Z_2$ est -CH = CH- et n = 1 ($BP_2$) peut être obtenue dans les mêmes conditions que ci-dessus en faisant réagir le composé de formule 6 avec le monoaldéhyde correspondant au composé de formule 7. Ce monoaldéhyde de départ peut être préparé selon la réaction de SOMMELET à partir de la monobromo-méthyl-bipyridine de formule 3 ($Z_2$ = $CH_2$ ; X = Br, $R_2$ = $CH_3$) par réaction avec l'hexaméthylènetétramine et hydrolyse du sel d'ammonium quaternaire ainsi formé à pH compris entre 3 et 6,5.

Pour l'obtention des oligobipyridines supérieures (n = 3 à 6) dans lesquelles -$Z_1$-Y-$Z_2$ est le groupe -CH=CH- on procède selon l'un des procédés décrits ci-dessus en préparant d'abord le dérivé monoaldéhyde de l'oligobipyridine $BP_2$ ou $BP_3$ et en le faisant réagir avec le dérivé monophosphonate 6 ou le dimère ou trimère correspondant manohalogéné, soit le diphosphonate correspondant.

Les oligobipyridines selon l'invention dans lesquelles -Z1-Y-$Z_2$- est -$CH_2$-$CH_2$- peuvent être obtenues par hydrogénation des oligobipyridines de formule I dans lesquelles -$Z_1$-Y-$Z_2$ est le groupe -CH=CH- selon des procédés classiques à la portée de l'homme de l'art.

L'obtention des composés de l'invention a été décrite en détail ci-dessus pour les composés de formule I dans laquelle $R_1$, $R_2$, $Z_1$, $Z_2$ sont en positions 6,6'. On notera que les oligobipyridines dans lesquelles les substituants $R_1$, $R_2$, $Z_1$, $Z_2$ sont en position 5,5' peuvent être obtenus selon les mêmes schémas réactionnels en utilisant comme composés de départ les analogues des composés de formule 1 à 7, c'est-à-dire les composés correspondants substitués en position 5,5'.

Dans le cas des oligobipyridines substituées ($R_3 \neq H$) on prépare d'abord les oligobipyridines substituées par le groupe t-butoxycarbonyle ($R_3 = COOC(CH_3)_3$) en suivant l'un des schémas réactionnels décrits ci-dessus, on les transforme ensuite selon des procédés classiques en les dérivés acides correspondants ($R_3$ = COOH), puis en les halogénures d'acides correspondants ($R_3$ = COX avec X = halogène, de préférence le chlore).

Par réaction de ces halogénures d'acides avec une base en présence de triéthylamine et sous atmosphère inerte, on peut obtenir les oligobipyridines de formule I dans laquelle $R_3$ est un groupe amido $CONR_7R_8$ tel que défini précédemment.

Les oligobipyridines de formule I dans laquelle $R_3$ est le groupe $COOC(CH_3)_3$ sont donc des intermédiaires-clefs pour l'obtention des oligobipyridines de formule I substituées par un groupe amido, tel que défini ci-dessus. Ces oligobipyridines substituées intermédiaires sont également des composés de l'invention qui présentent des propriétés complexantes.

Les composés de formules 1 à 4 dans lesquelles $R_3$ est le groupe $-COOC(CH_3)_3$ sont obtenus selon des procédés classiques à partir de la 4,4'-diméthoxycarbonyl-6,6'-diméthyl-2, 2'-bipyridine ou de la 4,4'-diméthoxycarbonyl-5,5'-diméthyl-2,2'-bipyridine ; leur préparation sera décrite en détail dans l'exemple 16 ci-après.

Les oligobipyridines selon l'invention permettent de complexer des ions métalliques, par exemple des ions cuivre, argent, lithium, etc.

Ces complexes sont obtenus en faisant réagir une solution de l'oligobipyridine dans un solvant approprié,tel que le chloroforme, avec unesolution d'un sel de l'ion métallique à complexer, par exemple le perchlorate ou le trifluoroacétate, dans un solvant approprié, tel que l'acétonitrile

La présente invention va maintenant être décrite plus en détail par les exemples non limitatifs ci-après, lesquels sont relatifs aux oligobipyridines $BP_2$, $BP_3$, $BP_4$ et $BP_5$ répondant à l'une des formules Ia à Ic ci-après :

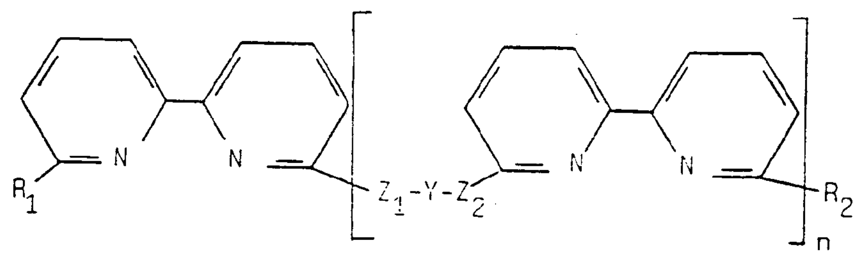

$I_a$

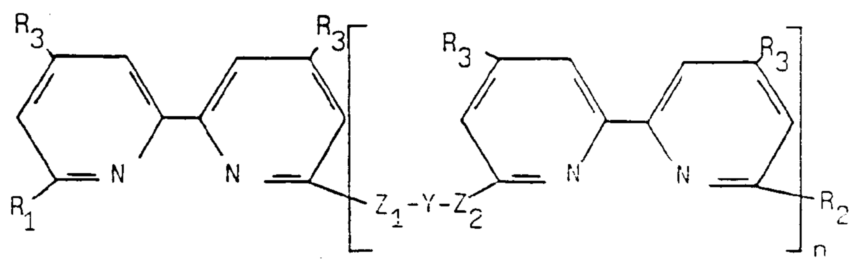

$I_b$

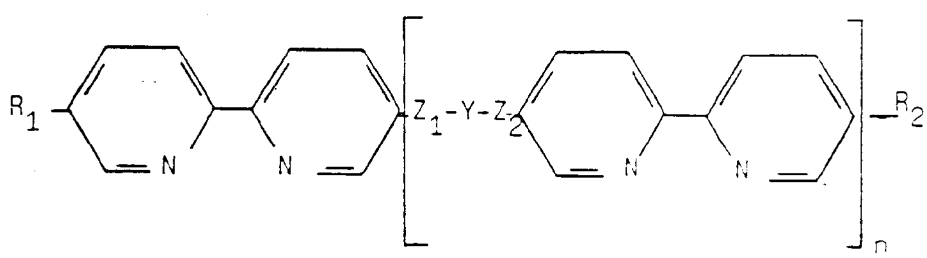

$I_c$

Exemple 1 : préparation d'une oligobipyridine $BP_2$

Formule Ia : $R_1 = R_2 = CH_3$ ; $Z_1 = Z_2 = -CH_2-$ ; n = 1 ; Y = 0

On a bromé de la 6,6'-diméthyl-2,2'-bipyridine avec du N-bromosuccinimide au reflux dans le $CCl_4$ en présence de peroxyde de benzoyle selon le mode opératoire décrit par Rodriguez Ubis et al. [Helv. Chim. Acta 67

2264-2269 (1984)].

Le dérivé monobromé ainsi obtenu a été traité avec une solution aqueuse de carbonate de sodium au reflux pendant 3 à 5 heures pour former le dérivé monohydroxylé 1 ($R_1$ = $CH_3$ et $Z_1$= $-CH_2-$) avec un rendement de 80 %.

Une solution du dérivé monohydroxylé 1 ainsi obtenu (400 mg , 2 mmoles) dans le tétrahydrofuranne (THF, 50 ml) a été traitée, sous argon, avec de l'hydrure de sodium à la température ambiante pendant 30 minutes. Ensuite on a ajouté un équivalent du dérivé monobromé 3 ($R_2$ = $CH_3$ ; X = Br et $Z_2$ = $-CH_2-$), préparé selon le mode opératoire indiqué ci-dessus, sous la forme solide et en une seule fois ; le mélange réactionnel résultant a été agité au reflux pendant 12 heures environ. Après évaporation du solvant, le résidu a été chauffé dans le méthanol au reflux pendant environ une heure et le mélange a été ensuite maintenu toute la nuit à 4°C. Le dépôt formé a été filtré et on a obtenu l'oligobipyridine $BP_2$ avec un rendement d'environ 60 %.

Ce produit a été purifié par chromatographie sur silice en utilisant le $CH_2Cl_2$ comme éluant. On a obtenu une poudre blanche dont le point de fusion est de 188-189°C.

Ce composé est peu soluble dans la plupart des solvants usuels, le chloroforme étant le meilleur solvant.

Exemple 2 : préparation de l'oligobipyridine $BP_3$

Formule Ia : $R_1$ = $R_2$ = $CH_3$ ; $Z_1$ = $Z_2$ = $-CH_2-$ ; n = 2 ; Y = 0

A une solution du dérivé monohydroxylé 1 obtenu selon l'exemple 1 (300 mg ; 1,5 mmole) dans le THF, refroidie à - 60°C (glace/méthanol) sous azote anhydre, on a ajouté goutte à goutte une solution (1,5 M) de butyllithium dans l'hexane jusqu'à ce que la couleur du mélange montre un changement brusque du jaune pâle au violet-marron. On a laissé ensuite la solution revenir à la température ambiante, puis on l'a traitée en une fois avec 0,3 équivalent du dérivé dibromé 4 (X = Br ; $Z_2$ = $-CH_2-$ ; 175 mg ; 0,5 mmole). Le mélange résultant a été ensuite agité pendant environ 18 heures.

Une précipitation importante de cristaux très fins blancs et brillants est alors intervenue. Après évaporation du THF sous vide, le produit a été transféré sur un filtre en utilisant du méthanol, puis lavé abondamment sur le filtre avec du méthanol, puis de l'éther et finalement séché à 100° C pendant 10 minutes (rendement : 247 mg ; 83 %).

Bien qu'à ce stade ce produit présente une pureté adéquate pour la préparation de complexes métalliques, on notera qu'il peut être ultérieurement recristallisé dans le chloroforme bouillant (50 ml/20 mg) par addition d'un volume égal d'acétonitrile et léger refroidissement. On obtient alors des cristaux fins incolores de $BP_3$ dont le point de fusion est de 227-229°C.

Comme le $BP_2$, cette oligobipyridine est peu soluble dans les solvants usuels, le chloroforme étant également le meilleur solvant pour le composé $BP_3$.

Exemple 3 : Préparation des complexes de cuivre (I) de ligands $BP_2$ et $BP_3$.

Les complexes de cuivre (I) de $BP_2$ et de $BP_3$ ont été obtenus par traitement d'une solution chloroformique du ligand (20 mM) par une solution dans l'acétonitrile contenant un léger excès d'un sel (perchlorate ou trifluoroacétate) de Cu(I). Ils ont également été préparés par addition d'une solution aqueuse de sel de Cu(II) à une suspension de $BP_2$ ou de $BP_3$ dans l'acétonitrile, suivie de réduction par l'hydrazine aqueuse. La couleur rouge orangé profond caractéristique des complexes Cu(I).bipyridine est apparue immédiatement. Il n'a pas été nécessaire d'effectuer la réaction sous argon, car les complexes résultants étaient stables à l'air. L'addition d'éther a permis de précipiter les complexes sous forme de solides rouges avec un rendement presque quantitatif. On les a redissous dans l'acétonitrile et on a ajouté une quantité égale d'eau. Les complexes $[Cu_2,(BP_2)_2]^{2+}$, $2CF_3COO^-$ et $[Cu_3,(BP_3)_2]^{3+}$, $3CF_3COO^-$ ont cristallisé lentement sous forme d'hydrates. Les trifluoroacétates obtenus étaient solubles dans une gamme de solvants beaucoup plus large ($CH_2Cl_2$, $CHCl_3$, $CH_3CN$, $CH_3OH$, acétone, éthanol, nitrobenzène, DMF, DMSO, eau) que les perchlorates. Ils ont donc été utilisés de préférence. Les nouveaux complexes obtenus avaient des propriétés spectrales et microanalytiques conformes à leur structure.

Exemple 4 : Détermination de la structure cristalline de $[Cu_3((BP_3)_2]$ $(CF_3COO)_3$.

On a fait pousser des cristaux du pentahydrate de ce complexe ($C_{78}H_{74}O_{15}N_{12}F_9Cu_3$, M = 1781,1) dans un mélange acétonitrile/eau. Ils appartiennent au groupe spatial monoclinique C/2c, a = 23,283(2), b = 19,614(2), c = 36,187(2) A,$\beta$ = 108,35° , V = 15685 Å, Z = 8. On a recueilli 5241 réflexions significatives ($I > 3\sigma_I$) sur un diffractomètre automatique Nonius CAD4 en utilisant la radiation K$\alpha$ de Cu et un cristal ayant approximativement les dimensions : 0,45 x 0,20 x 0,20 mm. La structure a été résolue en utilisant des techniques de Patterson et de Fourier. Tous les calculs ont été effectués avec l'ensemble des programmes Enraf-Nonius décrit par FRENZ B.A. dans "Computing in crystallography, eds. Schenk, H., Olthof-Hazekami, R., Van Konigsveld, H. & Bassi, G.C. (Delft Univ. Press) pp 64-71, (1978). A cause du désordre, les anions et les molécules d'eau n'ont pas pu être totalement localisés. L'affinement avec des facteurs de températures anisotropes pour le cation complexe (91 atomes) a conduit à un facteur R de 14 %. Le groupe étant à centre de symétrie (centrosymétrique), le cristal obtenu consiste en un mélange 1/1 d' hélices de chiralités opposées.

Exemple 5 : Formation des hélices à double brin polymétalliques

La bipyridine est un ligand chélatant bien connu qui forme des complexes avec la plupart des ions métalliques et elle a été étudiée de manière intensive à la fois pour ses propriétés de fixation et pour la photoactivité de plusieurs de ses complexes de métaux de transition.

On a observé la fixation de $Li^+$, $Ag^+$ et $Zn^{2+}$ par les variations induites dans le spectre de RMN protonique des ligands par l'addition d'un sel. On décrit ci-après la fixation de Cu(I) par les ligands obtenus selon les exemples 1 et 2 ci-dessus.

La fixation de Cu(I) par les ligands $BP_2$ et $BP_3$ a conduit immédiatement à la formation de la couleur rouge orangé profond caractéristique des motifs $[ Cu(I)(bipy)_2 ]^+$ et apparentés. Les complexes correspondants, isolés et caractérisés comme décrit ci-dessus, peuvent être représentés par les espèces dinucléaire $[Cu_2(BP_2)_2]^{2+}$ et trinucléaire $[ Cu_3(BP_3)_2 ]^{3+}$.

Nature des complexes de Cu(I) : hélices dinucléaire et trinucléaire à double brin.

Le complexe du ligand "dimère" $[Cu_2(BP)_2]^{2+}$ a une structure semblable à celle de $[ Cu_2(pQP)_2]^{2+}$, avec deux ions Cu(I) liés à deux groupes bipy, un de chaque molécule $BP_2$. Le complexe du ligand "trimère" $[Cu_3(BP_3)_3]^{3+}$ suit la même règle de structure et contient donc un double brin hélicoïdal de deux molécules de ligand enroulé autour de trois ions Cu(I) coordonnés "suivant un tétraèdre", comme représenté schématiquement sur la figure 1. Ces complexes peuvent être dénommés complexes en hélice ou hélices de Cu(I) à double brin, représentés par ds-$HM_n$, où n représente le nombre de centres métalliques M. Les coordinations des complexes de Cu(I) avec les composés de l'invention $BP_2$ et $BP_3$ sont analogues à la coordination pseudo-tétraédrale des complexes de Cu(I) de la 6,6-diméthyl- bipyridine et de la quaterpyridine pQP décrite par BURKE et al. dans Inorg. Chem. 19 (1980) 1211-1214 et par LEHN et al. dans Nouv. J. Chim. Z (1983), 413-420.Les complexes hélicoïdaux de ligands polyoxyéthylène sont du type à simple brin, ss-. Ces structures sont conformes aux propriétés physicochimiques des complexes et ont été confirmées par détermination de la structure cristalline du cation complexe $[Cu_3(BP_3)_2]^{3+}$ (voir ci-dessous).

Propriétés spectrales et physico-chimiques.

Les absorptions électroniques des deux complexes sont respectivement : $[Cu_2(BP_2)_2]^{2+}$ $\lambda_{max}$ : 449 nm, $\varepsilon$ = 9 800 dans $CHCl_3$, $[Cu_3(BP_3)_2/^{3+}$ $\lambda_{max}$ : 449 nm, $\varepsilon$ = 14 600 dans $CHCl_3$, elles correspondent à celles attendues pour de tels complexes de Cu(I)-bipyridine étant donné les caractéristiques connues du complexe [Cu(I), bis-6,6'-diméthylbipyridine ] qui sont : $\lambda_{max}$ = 454 nm, $\varepsilon$ = 6700) et celles du complexe dinucléaire de Cu(I) de la quaterpyridine pQP qui sont : $\lambda_{max}$ = 454 nm, $\varepsilon$ = 12700 (Voir article de LEHN et al. cité précédemment).

Les spectres de RMN protonique des ligands $BP_2$ et $BP_3$ sont nettement différents de ceux de leurs complexes de Cu(I). Les résonances des protons de la bipyridine sont déplacées par complexation, mais les variations les plus significatives se produisent pour les signaux de $CH_2OCH_2$. Tandis que ceux-ci sont des singulets dans les ligands libres, ils sont déplacés vers les champs croissants de plus de 1 ppm et scindés en deux diagrammes AB (J ~ 13 Hz) dans les complexes ; deux de ces diagrammes sont présents dans le spectre de $[Cu_3(BP_3)_2]^{3+}$ (Figure 2a : ligand libre ; Figure 2b : complexe). Donc, les protons de $CH_2$ sont devenus non équivalents dans les complexes, en raison de la perte du plan de symétrie correspondant. En outre, leur fort déplacement vers les champs élevés indique qu'ils se trouvent probablement maintenant dans la région de blindage (écran) des motifs bipyridines.

L'addition d'un excès du réactif chiral anthracényl-1-trifluorométhyléthanol à une solution de $[Cu_2(BP_2)_2 ]^{2+}$ dans $CD_2Cl_2$ produit une scission partielle du diagramme AB de $CH_2$ ; une scission mieux résolue a été obtenue pour les signaux de $CH_3$ du complexe $[Cu_2(pQP)_2]^{2+}$. Ces observations et la présence de diagrammes AB de $CH_2$ montrent que les complexes sont chiraux conformément à leur structure hélicoïdale.

Comme les diagrammes AB de $CH_2$ sont un diagnostic des complexes, la stabilité cinétique de ces derniers peut être testée. L'échange rapide des ligands (sur l'échelle temps de RMN) doit provoquer une conversion

$AB \rightarrow A_2$. Des études de RMN protonique à température variable du complexe dinucléaire [ $Cu_2(BP_2)_2$ ]$^{2+}$ (dans le nitrobenzène-$\underline{d_5}$) ont montré que les résonances AB de $CH_2$ (lente séparation d'échange d'environ 40 Hz à 200 MHz) se déplaçaient ensemble et s'élargissaient par chauffage, mais que la coalescence (fusion des pics) n'était pas atteinte à la température la plus élevée étudiée (393°K). Si ces changements résultent de l'échange des ligands et indiquent le début de la coalescence, la barrière à ce processus de "détorsion" de l'hélice doit être de plus d'environ 21 kcal/mole, la température de coalescence étant estimée à au moins 433°K . La barrière à cet échange doit même être supérieure pour le complexe trinucléaire $[Cu_3(BP_3)_2]^{3+}$ de sorte que la résolution chirale serait possible.

Des expériences de titrage par spectre de RMN et UV-visible ont été effectuées par addition progressive d'une solution de [ $Cu(I)(CH_3CN)_4$ ] $ClO_4$ dans l'acétonitrile à une solution de $BP_3$ dans le chloroforme. Seuls le ligand libre et son complexe trisCu(I) ont été observés dans toute la gamme de titrage, en quantités croissantes depuis 0,1 éq. de Cu(I). Ceci explique une "coopérativité positive dans la complexation de Cu(I), la fixation d'un ion facilitant probablement la fixation du suivant en raison d'une préorganisation des ligands.

Structure cristalline du complexe $[Cu_3(BP_3)_2]^{3+}3CF_3CO_2^-$ : une double hélice inorganique.

Afin de confirmer la structure attribuée aux complexes décrits ci-dessus et plus précisément de déterminer leurs paramètres géométriques, on a déterminé la structure cristalline du complexe trinucléaire $[Cu_3(BP_3)_2](CF_3COO)_3$ ; trois vues sont représentées sur la figure 3.

On voit que le cation $[Cu_3(BP_3)_2]^{3+}$ est bien entendu un complexe en hélice à double brin (figure 3a) correspondant à la représentation schématique donnée à la figure 1 : deux molécules du ligand $BP_3$ sont enroulées l'une autour de l'autre et maintenues ensemble par trois ions Cu(I), qui maintiennent la structure par interactions de coordination métallique comme la liaison hydrogène des paires de bases maintient la double hélice dans les acides nucléiques. L'image schématique de la figure 1 a la symétrie molèculaire $D_2$, conformément aux données de RMN en solution ; sur les trois axes de symétrie $C_2$, l'un passe à travers les trois ions cuivre, les deux autres sont perpendiculaires au premier à travers l'ion central, l'un se trouvant dans le plan de la figure et l'autre perpendiculaire à celui-ci. Ce n'est pas le cas pour la structure à l'état solide, comme indiqué par les figures 3b et 3c. Tandis que les plans de trois groupes bipyridine sont parallèles (figure 3b), ceux des trois autres ne le sont pas (angle d'inclinaison 27°) (figure 3c) ; en conséquence, un seul axe $C_2$ est conservé, celui perpendiculaire au plan de la figure 1. En outre, un processus de flexion-torsion dynamique de l'hélice doit être présent en solution, échangeant les trois bipyridines parallèles avec les trois autres et conférant une symétrie moléculaire moyenne $D_2$. Enfin, cet enroulement global de la double hélice possède des analogies avec celui rencontré dans l'ADN courbé (Saenger, W. (1984) principles of Nucleic Acid Structure (Springer-Verlag New-York , Heidelberg, Berlin) (Ulanovsky, L. et al. (1986) Proc. Natl. Acad. Sci. USA 83, 862-866) et avec le début d'une sur-torsion comme on la trouve dans l'ADN sur-enroulé selon Saenger (voir article cité ci-dessus).

Les caractéristiques hélicoïdales (figure 3a) de chaque brin dont définies par une longueur totale d'environ 16,5-17 Å (entre les groupes $CH_3$ terminaux) pour environ 1,5 tour, un pas (longueur par tour) d'environ 12 Å, un rayon d'environ 6 Å pour le cylindre circonscrit. Un tour de la double hélice contient deux centres d'interaction, deux ions métalliques. Les deux brins ont la même chiralité, c'est-à-dire le même sens d'hélice. En comparaison, la double hélice des acides nucléiques a un pas d'environ 30-35 Å, un rayon extérieur d'environ 10 Å et 10-11 interactions de paires de bases par tour (selon Saenger, voir article ci-dessus).

On peut aussi remarquer que les motifs à double brin formés par le $BP_2$ et le $BP_3$ (figure 1) ainsi que par la pQP ont les caractéristiques entrelacées de la "coupe duroi" (Anetet al., J. Am. Chem. Soc. 105, 1419-1426. et d'une tresse avec deux filaments et deux ou trois croisements (Walba, D.M. (1985) Tétrahédron 41, 3161-3212. La connection bout à bout des filaments de ce type de complexes dinucléaires peut permettre la synthèse d'un noeud moléculaire.

L'empilement des bases de bipyridine correspond à une distance de 3,6 Å entre les plans des bases parallèles (figure 3b). La distance la plus courte entre les bipyridines non parallèles (figure 3c) est d'environ 3,4 Å, comme on l'attend pour le contact selon Van der Waals et elle est semblable à la séparation des paires de bases empilées dans l'ADN selon Saengler (voir article cité précédemment). La déformation de l'hélice modifie nettement le recouvrement des bases empilées : tandis que les trois bases non parallèles se recouvrent de plus d'un noyau pyridine complet (bases à gauche de la figure 3b), le recouvrement des trois bipyridines parallèles est réduit à environ 1/3 d'un groupe pyridine (bases à gauche de la figure 3c). La flexion de la structure globale peut donc être due en partie à la tendance d'un ensemble de trois bipyridines à atteindre le contact selon Van der Waals et à se recouvrir ; une autre cause pourrait être la longueur des ponts $CH_2OCH_2$ séparant les motifs bipyridines. L'empilement des bipyridines, bien que seulement partiel, peut néanmoins contribuer à la stabilisation de la double hélice et favoriser son assemblage spontané à partir des composants.

Les propriétés de coordination des ions Cu(I), qui forment des complexes pseudo-tétraédraux avec des

ligands bipyridine et phénanthroline, induisent l'organisation des ligands oligobipyridines $BP_2$ et $BP_3$. Dans le présent complexe, les trois centres cuivre (I) sont presque identiques et ont une géométrie de coordination tétraédrale déformée. Les longueurs des liaisons Cu-N (moyenne de 2,02 Å) et les angles des liaisons N-Cu-N (2 de 82° et 4 entre 115 et 131°) sont comparables à ceux du complexe bis-6,6'-diméthylbipyridine-Cu(I)décrits par BURKE et al. dans Inorg. Chem. 19, (1980) 1211-1215. La même chose s'applique pour les angles des dièdres (environ 80°) entre les plans des deux motifs bipyridines à chaque centre Cu(I).

Exemple 6 : Préparation d'une oligobipyridine $BP_2$.

Formule Ia : $R_1 = CH_3$ ; $R_2 = CH_2Br$ ; $Z_1 = Z_2 = -CH_2-$ ;Y = 0 ; n = 1

0,900 g (4,5 mmoles) de la 6-méthyl-6'-hydroxyméthyl-2,2'-bipyridine (dérivé monohydroxylé 1 obtenu selon l'exemple 1) dans environ 50 ml de THF fraîchement distillé (sur sodium/ benzophénone) a été maintenue sous azote anhydre à la température ambiante.

On a ajouté en une fois 0,110 g de NaH (4,58 mmole) - 1,02 équivalent) et on a agité le mélange à la température ambiante pendant environ 1/2 heure.

La 6,6'-diméthyl-2,2'-bipyridine dibromée selon le mode opératoire décrit à l'exemple 1 a été alors ajouté en une fois et on a chauffé au reflux pendant 24 heures.

Le mélange réactionnel a été réduit à siccité puis repris dans environ 50 ml de $CH_2Cl_2$ et agité. On a filtré le solide résiduel qui a été recristallisé dans $CHCl_3$ à chaud. On a obtenu environ 15 % de trimère $BP_3$ (0,380 g).

Le filtrat a été concentré et chromatographié sous pression sur silice I, éluant

1) $CH_2Cl_2 \rightarrow$ bipyridine dibromée de départ (0,550 g ; 30 %) ;

2) 1 % $MeOH/CH_2Cl_2 \rightarrow BP_2$ attendu (0,720 g ; 35 %) ;

Le dimère monobromé ($BP_2$) a été trituré dans du méthanol à chaud, puis refroidi et filtré.

Le produit obtenu avait les caractéristiques suivantes :

– poids moléculaire : 461,4 ;
– point de fusion (banc kofler) : 154-156°C ;
– chromatographie couche mince sur $Al_2O_3$ ; 0,5 % $CH_3OH$ / $CH_2Cl_2$ : $R_f = 0{,}8$.

Exemple 7 : Préparation d'une oligobipyridine $BP_2$

Formule Ia : $R_1 = CH_3$ ;

$$R_2 = O-\underset{\underset{O}{\|}}{C}-CH_3 \;;$$

$Z_1 = Z_2 = -CH_2-$ ; n = 1; Y = 0

0,50 g (1,08 mole) de l'oligobipyridine obtenue selon l'exemple 6 ont été dissous dans environ 50 ml de DMF et chauffés à 60°C.

0,850 g d'acétate de sodium (10,36 mmoles - 9,6 équivalents) ont été ajoutés en une fois, puis on a chauffé le mélange à 140°C pendant 15 heures.

Le DMF a alors été distillé à sec sous vide (pompe à palettes). Le résidu a été repris dans de l'eau, puis filtré.

Le solide obtenu a été séché par distillateur azéotropique par l'éthanol, puis sur la pompe à palettes.

On a obtenu 430 mg de produit (90 % de rendement) dont les caractéristiques sont les suivantes :

. poids moléculaire : 440,5
. point de fusion : 150°C environ
. chromatographie couche mince sur $Al_2O_3$ ; 0,5 % $CH_3OH$ / $CH_2Cl_2$ : $R_f = 0{,}6$

Exemple 8 : Préparation d'une oligobipyridine $BP_2$

Formule Ia: $R_1 = CH_3$ ; $R_2 = CH_2OH$ ; $Z_1 = Z_2 = -CH_2-$ ; n = 1 ; Y = O

0,429 g (0,974 mmoles) de la bis(bipyridine) monoacétate obtenue selon l'exemple 7 a été dissoute dans environ 50 ml de méthanol. Le mélange obtenu a été porté au reflux jusqu'à dissolution totale.

On a ensuite ajouté lentement de la soude (NaOH ; 0,2 g soit 5 mmoles) dans 1,5 ml d'eau. On a agité le mélange résultant et on l'a chauffé pendant 1/2 heure.

Après refroidissement, un produit blanc a précipité ; il a été filtré et lavé à l'eau puis séché une nuit à la

pompe à vide.

On a obtenu 265 mg (rendement 70 %) du produit attendu dont les caractéristiques sont les suivantes :

. poids moléculaire : 398,5

. point de fusion (banc kofler) : 178°C

. chromatographie couche mince sur $Al_2O_3$ : 0,5 % $CH_3OH$ / $CH_2Cl_2$ : $R_f$ = 0,1

Analyse élémentaire :

$C_{24}H_{22}O_2N_4$ , 1/2 $H_2O$ - poids moléculaire : 407,5

| | | | |
|---|---|---|---|
| Calculé : | C 70,74 | H 5,69 | N 13,75 |
| Trouvé : | C 70,82 | H 5,47 | |

Exemple 9 : Préparation d'une oligobipyridine $BP_4$

Formule Ia : $R_1 = R_2 = -CH_3$ ; $Z_1 = Z_2 = -CH_2-$ ; n = 3 ; Y = 0 ;

Le dérivé monohydroxylé 1 a été mis à réagir avec un équivalent de NaH dans du THF à la température ambiante, pendant 30 min. L'addition d'un équivalent du dérivé dibromé 4 (X = Br ; $Z_2 = CH_2$) et chauffage au reflux pendant 3 heures a donné le dérivé dimère monobromé 8 répondant à la formule :

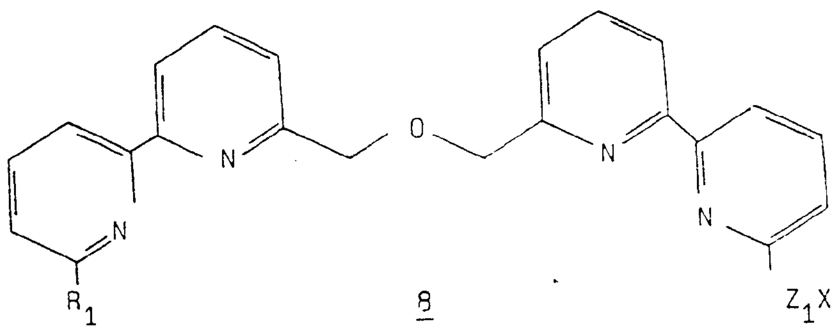

8

Ce dérivé monobromé 8 est séparé du mélange réactionnel par chromatographie sur silice avec comme éluant le mélange MeOH / $CH_2Cl_2$ : 1/99. La poudre obtenue a une température de fusion de 152 - 155° C.

Le dimère monobromé 8 est mis à réagir avec un grand excès d'acétate de sodium dans le DMF à 140° C pendant 12 heures ; l'hydrolyse du dimère monoacétate obtenu (NaOH/MeOH/$H_2O$ - reflux 1/2 h) donne le dimère monoalcool 9 correspondant ($R_1 = CH_3$ ; $Z_1 = CH_2$ ; X = OH).

Le traitement de ce dimère monoalcool 9 par le tertiobutylate de potassium dans THF en présence de quelques gouttes de DMSO à température ambiante pendant 1/2 h, suivi de l'addition d'un équivalent du dérivé monobromé 8 et chauffage au reflux pendant deux heures donne le composé $BP_4$ sous la forme d'un précipité blanc qui est séparé du mélange réactionnel par filtration et lavé avec $CH_2Cl_2$ (rendement : 68 % ; point de fusion > 260° C).

Exemple 10 : Préparation d'une oligobipyridine $BP_5$

Formule Ia : $R_1 = R_2 = -CH_3$ ; $Z_1 = Z_2 = -CH_2-$ ; Y = 0 ; n = 4

Dans environ 50 mlde THF on a ajouté 0,130g (0,326 mmole) de l'oligobipyridine préparée selon l'exemple 8 (correspondant au dérivé monohydroxylé de la $BP_2$ de l'exemple 1) puis on a ajouté en une fois 0,045 g (0,401 mmole) de tertiobutylate de potassium (t-BuOK) et quelques gouttes de DMSO. On a ensuite agité à température ambiante pendant 1/2 heure.

On a ensuite ajouté en une seule fois 0,056 g (0,164 mmole) du dérivé dibromé 4 préparé selon l'exemple 2. Le mélange obtenu a été chauffé au reflux pendant 16 heures ; il s'est éclairci de plus en plus jusqu'à la couleur jaune pâle.

Le solide blanc en suspension a été filtré et lavé a l'eau puis à l'éthanol et enfin séché à la pompe à palette.

On a obtenu 50 mg de la $BP_5$ attendue (rendement 68 %).

Analyse élémentaire :

$C_{60}H_{50}O_4N_{10}$, $3H_2O$ ; poids moléculaire : 1 031,15

| | | | |
|---|---|---|---|
| Calculé : | C 69,88 | H 5,67 | N 13,58 |
| Trouvé : | C 69,74 | H 5,20 | N 12,97 |

Point de fusion (banc kofler) : > 260° C.

Exemple 11 : Préparation des complexes de Cu(I) de $BP_4$ et $BP_5$

Les complexes de Cu (I) de $BP_4$ et $BP_5$ ont été obtenus par traitement d'une suspension du ligand $BP_4$ ou $BP_5$ préparé selon les exemples 9 au 10 dans de l'acétonitrile par une solution dans l'acétonitrile contenant un léger excès d'un sel de cuivre (II), à savoir le trifluorométhanesulfonate ($CF_3SO_3^-$).

La solution verte obtenue est traitée par un léger excès d'hydrazine hydratée. La couleur rouge caractéristique des complexes [Cu(I) bipy] est apparue immédiatement.

Les complexes de Cu (I) de $BP_4$ et $BP_5$ ont une structure analogue à celle des complexes de Cu (I) de $BP_2$ et $BP_3$, c'est-à-dire une structure en hélice (voir exemple 5 ci-dessus). Le complexe Cu (I) de $BP_5$, à savoir $[Cu_5(BP_5)_2]^{5+}$, est représenté schématiquement sur la figure 1 annexée.

Le spectre RMN 1 H dans $CH_3CN$ de ce complexe (Figure 4b) montre un déplacement vers les champs croissants de plus de 1 ppm des $CH_2$ qui deviennent non équivalents et scindés en diagramme AB par comparaison au spectre de l'oligobipyridine libre (Figure 4a).

Exemple 14 : Préparation d'une oligobipyridine pontée par des doubles liaisons

Formule Ia : $R_1 = R_2 = CH_3$ ; $-Z_1-Y-Z_2- = -CH = CH-$ ; n = 2

A - Préparation de la 4,4'-dibromobipyridine

Ce composé a été préparé en suivant le mode opératoire décrit dans J. Organometallic Chemistry 56 (1973) 53-66. 59,22 g (0,25 mole) de 2,6-dibromopyridine ont été solubilisés dans 476 ml d'éther éthylique anhydre refroidi à - 90° C sous argon ou azote.

156,3 ml de n-butyl-lithium 1,6 N dans de l'hexane ont été additionnés lentement, de façon à ce que la température du mélange réactionnel n'excède pas - 60° C.

Après l'addition, on a laissé la température remonter lentement jusqu'à - 40° C et on l'y a maintenu pendant 1/2 heure pour permettre la formation du dérivé lithien.

Le mélange réactionnel a été à nouveau refroidi à - 90° C et 16,81 g (0,125 mole ; 0,5 équivalent) de $CuCl_2$ ont été ajoutés en une seule fois sous forme solide.

La température a été portée à -50° C pendant 2 à 3 heures. Le mélange réactionnel est alors devenu brun noir.

Après ce temps, l'arrivée d'argon a été stoppée et on a fait buller de l'air comprimé jusqu'à l'obtention d'une coloration jaune.

On a laissé le mélange réactionnel revenir à la température ambiante, puis on l'a acidifié avec 250 ml de HCl 6N et on a agité pendant 1/2 heure.

Le solide vert-jaune qui a précipité a été filtré, lavé par HCl 1N, puis recristallisé dans du benzène.

On a obtenu 17 g du composé recherché (rendement 44 % ; point de fusion 216 - 218° C).

B - Préparation de la bipyridine 4,4'-dialdéhyde (formule 7)

Ce composé a été également préparé en suivant le mode opératoire décrit dans J. Organometallic Chemistry 56 (1973) 53-66.

16,7 g de n-butyl-lithium (26,7 mmole) ont été ajoutés à la seringue à 150 ml de THF anhydre refroidi à - 85° C, puis on a agité pendant 15 min. On a ensuite ajouté goutte à goutte 3,9 g du 4,4'-dibromo-bipyridine préparé précédemment (12,42 mmole) dans 160 ml de THF anhydre. La température du mélange réactionnel a été maintenue inférieure à - 70° C.

3,23 ml de diméthylformamide (41,5 mmole) dans 40 ml d'éther éthylique absolu ont ensuite été ajoutés goutte à goutte. Le mélange réactionnel vert foncé a été agité pendant 1 heure à température inférieure à - 70° C, puis pendant 1 heure à la température de - 30° C.

43 ml de HCl 6N ont été ajoutés au mélange réactionnel en 20 minutes. Le mélange brun clair obtenu a été rechauffé jusqu'à la température ambiante, puis agité à l'air pendant 1/2 heure.

Les solvants organiques ont été évaporés. La phase aqueuse résiduelle a été alcalinisée jusqu'à pH > 13 par NaOH 4N.

Le produit attendu a précipité ; il a été filtré, lavé à l'eau, séché et recristallisé dans du toluène (700 mg ; rendement : 30 %).

C - Préparation du phosphonate (formule 6)

0,53 g (0,2 mmole)de 6-monobromométhylène-bipyridine 5 et 5 ml (2,9 mmole) de triéthylphosphite ont été agités à la température ambiante sous argon pendant 1/2 heure.

Après dissolution, le mélange réactionnel a été chauffé à 150° C pendant 5 heures sous argon.

L'excès de triéthylphosphite a été distillé après refroidissement. L'huile obtenue a été chromatographiée sur silice avec $CH_2Cl_2$ comme éluant. Le produit attendu a été séparé sous la forme d'un solide jaune pâle (600 mg ; rendement 94 %).

D - Préparation de l'oligobipyridine

65 mg de NaH/huile à 55 % ont été ajoutés à 10 ml de THF anhydre sous argon à température ambiante.

Après 15 minutes d'agitation, le phosohonate 5 (380 mg ; 1,18 mmole) dans 5 ml de THF anhydre a été ajouté goutte à goutte (temps de l'addition : 15 min). L'agitation a été maintenue 15 minutes, puis le mélange réactionnel est devenu orange.

125 mg (0,59 mmole ; 0,5 équivalent) du dialdéhyde 7 dans 50 ml de THF anhydre ont été additionnés lentement. L'agitation a été maintenue à température ambiante pendant 3 heures, pendant lesquelles le mélange réactionnel s'est éclairci.

On l'a alors chauffé au reflux (60° C) pendant 20 heures sous argon. Il s'est formé un précipité jaune.

Après addition de quelques millilitres d'eau, le produit désiré a été filtré, lavé avec du THF et séché.

On a obtenu 160 mg de l'oligobipyridine (rendement : 50 %).

Exemple 15 : Préparation d'une oligobipyridine $BP_2$ tétrasubstituée

Formule Ib : $R_1 = R_2 = CH_3$ ; $R_3 = COR_5$ avec $R_5 = OC(CH_3)_3$ ; n = 1 Y = 0.

39 mg ($9,7\ 10^{-5}$ mole) du composé monohydroxylé 1 ($R_1 = CH_3$ ; $Z_1 = -CH_2-$ ; $R_3 = -COOC(CH_3)_3$) ont été dissous dans 3 ml de THF fraîchement distillé sous azote et traités par 1,5 équivalents de NaH/huile ; le mélange obtenu a été agité à 40° C pendant 1/2 heure et est devenu jaune-vert.

Le dérivé monobromé 3 ($R_2 = CH_3$ ; $R_3 = -COOC(CH_3)_3$ ; X = Br et $Z_2 = CH_2$-) a alors été ajouté en une fois, sous forme solide On a laissé la réaction se poursuivre sous azote à 40° C pendant 12 heures.

Après addition de quelques gouttes d'eau, le THF a été évaporé, puis le résidu a été repris dans $H_2O/CH_2Cl_2$ : 1/1. La phase organique a été soutirée, séchée sur sulfate de magnésium et concentrée après filtration.

L'oligobipyridine tétrasubstituée a été séparée par plaque préparatoire $Al_2O_3$, éluant $CH_2Cl_2$:hexane : 1/1. On a obtenu 30 mg de produit (rendement : 40 %).

Exemple 16 : Préparation d'un oligobipyridine substituée

Formule Ib : $R_1 = R_2 = CH_3$ ; $R_3 = -COOC(CH_3)_3$; n = 2 $Z_1 = Z_2 = -CH_2-$ ; Y = 0

A - Préparation de la 4, 4'-di-t-butoxycarbonyl-6, 6' -diméthyl-2,2'- bipyridine.

On a ajouté, goutte à goutte et sous atmosphère d'azote, une solution de t-butanolate de lithium, préparée à partir de n-butyllithium (40 ml) et de t-butanol (150 ml) selon le procédé décrit dans Org. Synth. 1971, 51, 96, à une solution de 4,4'-diméthoxycarbonyl-6,6'-diméthyl-2,2' bipyridine (4,0 g ; 0,013 mole) dans du toluène (500 ml) à 80° C.

Lorsque l'addition a été terminée, la solution a été agitée pendant 30 minutes à 80° C, puis refroidie et trempée dans l'eau. On a gardé la phase organique, on l'a lavée à l'eau trois fois, puis séchée sur $MgSO_4$ et on a évaporé le solvant ; on a obtenu le produit brut sous la forme d'un solide jaune pâle (4, 83 g, 94 %).La recristallisation dans le mélange $CHCl_3/MeOH$ : 1/10 a donné la 4,4'-di-t-butoxycarbonyl-6,6'-diméthyl-2,2'-bipyridine sous forme d'aiguilles blanches (4,6 g ; 90 %), dont l'analyse élémentaire est la suivante :

$C_{22}H_{28}O_4N_4$ : poids moléculaire 384,46

calculé : C : 68,72 ; H : 7,34 ; N : 7,29

trouvé : C : 68,33 ; H ; 7,41 ; N : 7,24

Dans les deux étapes ci-après, on a suivi les modes opératoires décrits par G.R. Newkome et al dans J. Org. Chem. 1982, 47, 4116-4120.

B - Préparation des dérivés mono- et di-bromés de formules 3 et 4

Formule 3 : $R_2$ = - $CH_3$ ; $R_3$ = - $COOC(CH_3)_3$ ; $Z_2$ = - $CH_2$- ; X = Br
Formule 4 : $R_3$ = - $COOC(CH_3)_3$ ; $Z_2$ = - $CH_2$ - ; X = Br.

On a dissous dans du benzène (200 ml) du 4,4′-di-t-butoxycarbonyl-6,6′-diméthyl-2,2′-bipyridine (3,0 g ; 7,8 mmole), du N-bromosuccinimide (5,6 g ; 31,3 mmole) et du AIBN (100 mg). On a chauffé ce mélange au reflux sous atmosphère d'azote pendant 24 heures. Au bout de dix heures, on a rajouté du N-bromosuccinimide (3,0 g ; 17 mmole) au mélange réactionnel.

On a refroidi la solution, on a évaporé le solvant et le solide résiduel a été chromatographié sur gel de silice ($CH_2Cl_2$). Les produits tétra-et tri-bromés ont d'abord été élués, puis ensuite le mélange des deux produits dibromés (3, 27g) et la 4,4′-di-t-butoxycarbonyl-6-méthyl-6′-bromométhyl-2,2′-bipyridine (composé de formule 3 (0,7 g ; 19 %) qui a recristallisé sous forme d'aiguilles blanches dans un mélange $CHCl_3$/MeOH. Les deux produits dibromés ont été séparés par chromatographie radiale sur silice (2 % acétate d'éthyle/hexane, le produit étant chargé dans le benzène). La 4,4′-di-t-butoxycarbonyl-6-méthyl-6′ dibromométhyl-2, 2′-bipyridine a élué en premier, puis ensuite la 4,4′-di-t-butoxycarbonyl-6,6′-dibromométhyl-2,2′-bipyridine (composé de formule 4), qui après évaporation du solvant, a été obtenue sous la forme d'un solide blanc duveteux (1,3 g ; 31 %).

L'analyse élémentaire des composés obtenus est la suivante :

Composé de formule 3 : $C_{22}H_{27}O_4N_2Br$ poids moléculaire 463,36

calculé : C 57,02 ; H 5,87 ; N 6,05

trouvé : C 56,86 ; H 5,80 ; N 5,99

Composé de formule 4 : $C_{22}H_{26}O_4N_2Br_2$ poids moléculaire 542,25

calculé : C 48,73 ; H 5,17 ; N 4,83

trouvé : C 48,95 ; H 5,19 ; N 4,83

C - Préparation de la 4,4′-di-t-butoxycarbonyl-6-méthyl- 6′-acétoxyméthyl-2,2′-bipyridine

On a versé goutte à goutte une solution de 4,4′-di-t-butoxycarbonyl-6,6′-diméthyl-2,2′-bipyridine (2,0g ; 6,7 mmole) dans une solution d'acide m-chloroperbenzoïque (1,0 g ; 85 %, 6,8 mmole). La solution a été maintenue sous agitation, à 0°C pendant 3 heures et ensuite à température ambiante pendant 5 heures. On a lavé la phase organique avec une solution aqueuse saturée de $NaHCO_3$, et de l'eau, on l'a séchée sur $MgSO_4$ et on a évaporé le solvant pour donner le mono-N-oxyde brut sous forme d'un solide jaune (2,16g). En général, le produit brut a été utilisé tel quel dans l'étape suivante sans purification. Le produit pur peut être isolé par chromatographie sur silice avec le MeOH/$CHCl_3$) à 1 % qui élue les produits de départ qui n'ont pas réagi, puis le mono-N-oxyde et une petite partie du di-N-oxyde. La recristallisation dans le mélange $CHCl_3$/hexane donne le 4,4′-di-t-butoxycarbonyl-6,6′-diméthyl-2,2′-bipyridine N oxyde sous forme d'aiguilles blanches.

Le produit brut (2,16 g) a été chauffé au reflux dans de l'anhydride acétique (50 ml) pendant 30 min. On a évaporé à sec la solution résultante, marron foncé, et le résidu a été repris dans $CHCl_3$. La solution a été lavée deux fois avec une solution aqueuse saturée de $NaHCO_3$, puis avec de l'eau ; elle a été ensuite séchée sur $MgSO_4$ et le solvant a été évaporé. On a effectué la purification par chromatographie flash sur silice avec ($CHCl_3$), comme éluant et on a obtenu la 4,4′-di-t-butoxycarbonyl-6-méthyl-6′-acétoxyméthyl 2,2′-bipyridine sous forme d'un solide jaune pâle (1,85 g, 80 % ; poids moléculaire 443,50).

D - Préparation de la 4,4′-di-t-butoxycarbonyl-6-méthyl-6′-(hydroxyméthyl-2,2′-bipyridine

Formule 1 : $R_1 = CH_3$ ; $R_3$= $-COOC(CH_3)_3$ ; $Z_1 = -CH_2-$

On a dissous 1,8 g (17 mmole) de carbonate de sodium dans l'eau (30 ml) et on l'a ajouté à une solution, sous agitation, de 4,4′-di-t-butoxycarbonyl-6-méthyl-6′-acétoxyméthyl-2, 2′-bipyridine(1,5 g ; 3, 4 mmole) dissous dans 100 ml de THF. La solution a été chauffée à reflux toute la nuit, on a évaporé le THF et on a extrait le produit dans du $CH_2Cl_2$. Les couches organiques combinées ont été séchées sur $MgSO_4$, le solvant a été éliminé et on a obtenu, par recristallisation du solide restant dans un mélange $CHCl_3$/hexane, le 4,4′-di-t-butoxycarbonyl-6-méthyl-6′-hydroxyméthyl-2,2′-bipyridine (1,05 g; 78 %).

analyse élémentaire : $C_{22}H_{28}O_5N_2$ poids moléculaire 400,5

| | | | |
|---|---|---|---|
| calculé : | C 65,98 | H 7,05 | N 7,00 |
| trouvé : | C 65,87 | H 6,97 | N 7,00 |

E - Préparation de l'oligobipyridine substituée

On a chauffé à 60° C sous atmosphère d'azote de la 4,4′-di-t-butoxy-6′-méthyl-6-hydroxyméthyl-2,2′-bipyridine (composé de formule 1 ; 487 mg, 1, 22 mmole) dans le THF avec de l'hydrure de sodium (64 mg ; dispersion à 55 % dans l'huile). 45 minutes plus tard, on a versé goutte à goutte dans la solution devenue jaune foncé, une solution de 4,4′-di-t-butoxy-6, 6′-dibromométhyl-2,2′-bipyridine (300 mg ;0,55 mmole) dissous dans 10 ml de THF. On a chauffé la solution au reflux pendant 22 heures, puis on l'a refroidie, on a ajouté de l'eau et on a évaporé le THF. Le produit a été extrait par du $CH_2Cl_2$, lavé à l'eau, séché sur $MgSO_4$ et le solvant a été éliminé pour donner le produit brut. La recristallisation dans le $CH_2Cl_2$ a donné un solide blanc qui était l'oligobipyridine attendue à l'état pur (92 mg). La liqueur mère a été évaporée jusqu'à siccité et soumise à une chromatographie flash sur silice ($CHCl_3$). On a obtenu encore 155 mg de l'oligobipyridine pure (rendement total 247 mg, 38 %).

Analyse élémentaire :

$C_{66}H_{80}N_6O_{14}$ poids moléculaire 1 181,4

| | | | |
|---|---|---|---|
| calculé : | C 67,10 ; | H 6,83 ; | N 7,11 |
| trouvé : | C 67,19 ; | H 6,67 | |

Exemple 17 : Préparation d'une oligobipyridine substituée par un groupe amido

Formule Ib : $R_1 = R_2 = CH_3$ ; $R_3$ = CONH-5′-déoxyadénosine $Z_1 = Z_2 = -CH_2-$ ; Y = 0 ; n = 2

A - Préparation de l'oligobipyridine hexa-acide et de l'oligobipyridine hexachlorure

($R_3$ = COOH ou COCl)

On a agité pendant une heure l'ester d'hexa-t-butyle (67 mg 0,06 mmole) dans une solution aqueuse d'HCl (5 ml, 5M). On a versé le tout sur de la glace et on a ajusté le pH jusqu'à 2-3 avec une solution de NaOH saturée. On a filtré l'hexa-acide résultant, on l'a lavé à l'eau et on l'a séché pour obtenir un rendement quantitatif d'acide pur.

L'acide a été chauffé à reflux avec 10 ml de chlorure de thionyle sous atmosphère d'azote. Quand l'acide a été entièrement dissous, la solution jaune pâle a été chauffée pendant 1 heure de plus. On a évaporé le solvant, on a séché sous vide (8 heures) et on a obtenu l'oligobipyridine hexachlorure sous forme d'un solide jaune pâle dans un rendement quantitatif.

B - Préparation de l'oligobipyridine

On a ajouté goutte à goutte sous atmosphère d'azote, une solution de chlorure d'hexa-acide (23 mg ; 0,023 mmole) dissous dans $CH_2Cl_2$ (6 ml) à une solution mise sous agitation de 5′-amino-5′ deoxyadénosine (41 mg ; 0,153 mmole) et de triéthylamine (6 gouttes) dissout dans du t-butanol (60 ml). La solution a été agitée à température ambiante pendant 4 heures durant lesquelles un précipité s'est formé. On a attendu 40 heures, puis

on a filtré le précipité d'hexa-amide brut et on l'a lavé avec $CHCl_3$. Le solide a été purifié par ébullition dans MeOH (50 ml) pendant 30 minutes avant filtration. Le solide insoluble a été traité de la même manière dans $CH_3CN$, filtré et séché pour donner un solide crémeux (13 mg, 24 %) qui est de l'oligobipyridine substituée ($R_3$ =CONH-5'-déoxyadénosine) à l'état pur ($C_{102}H_{104}N_{42}O_{26}$ ; poids moléculaire : 2334,15).

Exemple 18 : Préparation d'une oligobipyridine substituée par un groupe amido

Formule Ib : $R_1 = R_2 = CH_3$ ; $R_3$ = CO-NH-5'déoxythymidine $Z_1 = Z_2 = -CH_2-$ ; Y = 0 ; n = 2

On a ajouté goutte à goutte pendant une heure une solution de l'oligobipyridine hexachlorure préparé selon le point A de l'exemple précédent (30 mg ; 0,03/mmole) disssoute dans $CH_2Cl_2$ (15 ml) à une solution mise sous agitation de 5'-amino-5'-déoxythymidine (55 mg ; 0,23 mmole) et de triéthylamine (5 gouttes) dissoute dans le t-butanol (30 ml) sous atmosphère d'azote. La solution a été agitée pendant 24 heures à la température ambiante et on a ensuite évaporé le solvant. Le solide résiduel a été lavé avec $CHCl_3$ puis chauffé à reflux dans du MeOH (15 ml) pendant 1 heure. Le solide insoluble obtenu par filtration a été chauffé au reflux dans $CH_3CN$ pendant 1 heure et refiltré. Le solide ainsi obtenu a été séché pour donner un solide crémeux qui est l'oligobipyridine attendue (29 mg ; 43 % ; $C_{104}H_{110}N_{24}O_{32}$ ; poids moléculaire : 2208,09).

Exemple 19 : Complexes de Cu(I) des oligobipyridines pontées ou substituées

Les complexes de Cu(I) des oligobipyridines obtenues, selon les exemples 14 à 18 ont été préparés, selon le mode opératoire décrit à l'exemple 13. Dans chaque cas, on a obtenu la couleur rouge caractéristique des complexes $[Cu(I)(bipy)_2]^+$.

Exemple 20 : Préparation de l'oligobipyridine de formule Ic

$R_1 = R_2 = CH_3$ ; $Z_1 = Z_2 = -CH_2-$ ; Y = 0 ; n = 2

100 mg de la 5-méthyl-5'-hydroxyméthyl-2,2'-bipyridine dans 15 ml de THF contenant 15 gouttes de DMSO anhydre, sont mis à réagir, à température ambiante et sous argon, avec 62 mg de tertiobutylate de potassium.

Lorsque l'alcoolate a été formé (la solution est devenue brun-vert) on a ajouté goutte à goutte 0,5 équivalent du 5,5'-dibromométhyl-2,2'-bipyridine (84,5 mg) dans 15 ml de THF anhydre.

Après l'addition le mélange a été porté à reflux pendant 15 heures, puis tiré à sec. Le résidu a été trituré à l'eau et filtré.

Le solide obtenu a été séché. On a obtenu 77 mg du produit désiré (rendement 53 %).

Exemple 21 : Préparation d'une oligobipyridine de formule Ia :

$R_1 = R_2 = CH_3$ ; $-Z_1-Y-Z_2- = -CH_2-CH_2-$ ; n = 2

Dans un tricol muni d'un ballon d'hydrogène, d'une sortie reliée au vide et d'un ballon d'argon, on a mis 10 mg du composé préparé à l'exemple 14, 8 ml d'éthanol absolu et 3 gouttes de HCl concentré.

On a ajouté 6 mg de Pd/C 10% et on a dégazé trois fois la solution par vide/argon.

Le mélange a été ensuite traité par l'hydrogène à température ambiante pendant 22 h, puis a été filtré sur célite.

Le filtrat a été essoré à siccité, repris dans 15 ml d'eau et basifié par NaOH 4N jusqu'à pH = 10.

Le produit désiré a été extrait au chloroforme, puis purifié par chromatographie sur alumine éluant $CH_2Cl_2$.

On a obtenu 7 mg de produit désiré pur (rendement 69 %).

## Revendications

1. A titre de nouveaux produits les oligobipyridines répondant à la formule I ci-après :

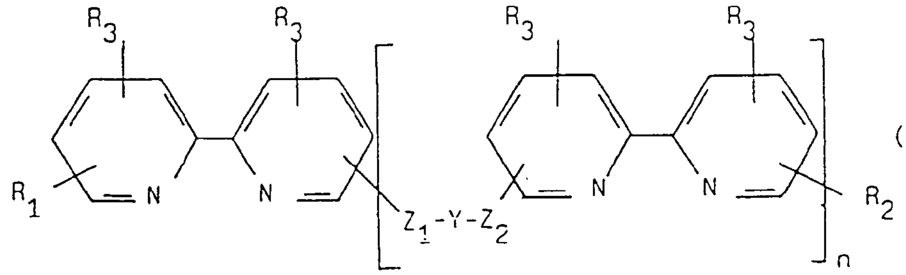

(I)

dans laquelle :

– $Z_1$ et $Z_2$, identiques ou différents, représentent des groupes alkylène en $C_1$ - $C_{12}$;

– Y est l'oxygène ou le soufre ou bien ;

– $Z_1$-Y-$Z_2$- représente les groupes -CH=CH- ou -$CH_2$ - $CH_2$

– n est un nombre entier compris entre 1 et 6 ;

– $R_1$ et $R_2$, identiques ou différents, représentent des groupes alkyle en $C_1$ - $C_5$ en particulier le groupe méthyle ou le groupe éthyle, les radicaux-$Z_1$YH, -$Z_2$YH, -$Z_1$X ou -$Z_2$X dans lesquels Y, $Z_1$ et $Z_2$ sont tels que définis ci-dessus et X est un atome d'halogène ou un groupe de formule

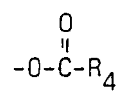

dans laquelle $R_4$ est un groupe alkyle en $C_1$ - $C_5$.

– $R_3$ est l'hydrogène ou un groupe -$COR_5$ dans lequel $R_5$ représente

. un halogène, de préférence le chlore ;

. un groupe $OR_5$ ou $R_5$ représente l'hydrogène, ou un groupe alkyle en $C_1$ - $C_5$ ; ou

. un groupe $NR_7R_8$ dans lequel $R_7$ et $R_8$, identiques ou différents, représentent l'hydrogène ou un groupe alkyle en $C_1$ - $C_5$ ou bien l'un d'eux représente l'hydrogène et l'autre est un nucléoside.

2. Composé selon la revendication 1, caractérisé en ce que $Z_1$, $Z_2$, $R_1$, $R_2$ sont en position 6,6′ ou 5,5′.

3. Composé selon l'une des revendications 1 ou 2, caractérisé en ce que les radicaux $R_3$ sont en positions 4,4′.

4. Composé selon l'une quelconque des revendications 1 à 3, caractérisé en ce que $Z_1$ et $Z_2$ sont le groupe -$CH_2$-, Y est l'oxygène et $R_1$ et $R_2$ sont identiques et représentent le groupe méthyle.

5. Composé selon l'une quelconque des revendications 1 à 3, caractérisé en ce que $Z_1$ et $Z_2$ sont identiques et représentent le groupe -$CH_2$-, Y est l'oxygène, $R_1$ est le groupe méthyle et $R_2$ est l'un des groupes ci-après:

$-CH_2Br$, $-CH_2OH$ ou 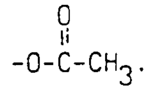

6. Composé selon l'une quelconque des revendications 1 à 5, caractérisé en ce que $R_3$ est l'hydrogène et en ce que n vaut 1, 2, 3 ou 4.

7. Composé selon la revendication 1, caractérisé en ce que -$Z_1$-Y-$Z_2$- est le groupe -CH=CH- ou le groupe -$CH_2$-$CH_2$.

8. Composé selon la revendication 7, caractérisé en ce que $R_3$ est l'hydrogène, $R_1$ et $R_2$ sont identiques et représentent le groupe méthyle.

9. Composé selon la revendication 3, caractérisé en ce que $R_3$ est le groupe -$COR_5$ dans lequel $R_5$ est le chlore, le groupe $OR_5$, $R_5$ représentant le groupe t-butyle ou l'hydrogène.

10. Composé selon la revendication 9, caractérisé en ce que $R_3$ est le groupe -$NR_7R_8$ dans lequel $R_7$ est l'hydrogène et $R_8$ est un nucléoside.

11. Composé selon la revendication 10, caractérisé en ce que $R_8$ est le reste de 5′-déoxyadénosine.

12. Composé selon la revendication 10, caractérisé en ce que $R_8$ est le reste de 5′-déoxythymidine.

13. Application des oligobipyridines selon l'une quelconque des revendications 1 à 12, à titre d'agents complexants d'ions métalliques.

14. Procédé pour l'obtention des oligobipyridines selon la revendication 1, répondant à la formule I dans

laquelle $Z_1$ et $Z_2$ sont un groupe alkylène, Y est l'oxygène ou le soufre et n = 1 ou 2 caractérisé en ce qu'il consiste à faire réagir au reflux le composé de formule 2

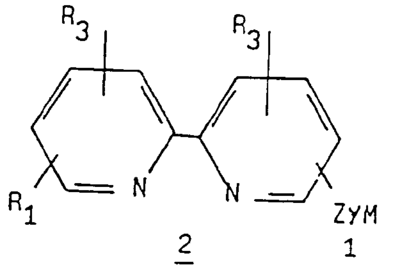

2

dans laquelle $R_1$, $R_3$, $Z_1$ et Y sont tels que définis dans la revendication 1 et M est un métal avec l'un des composés de formules 3 ou 4.

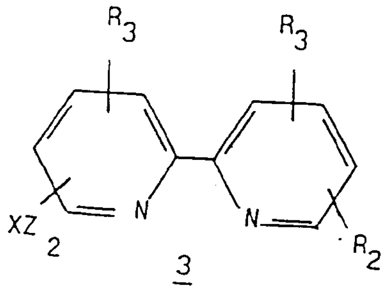

3

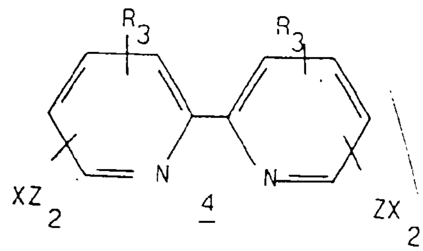

4

dans lesquelles $R_2$, $R_3$, $Z_2$ et X sont tels que définis dans la revendication 1 et à récupérer l'oligobipyridine ainsi formée.

15. Procédé pour l'obtention des oligobipyridines selon la revendication 1, répondant à la formule I dans laquelle $Z_1$ et $Z_2$ sont un groupe alkylène, Y est l'oxygène ou le soufre et n = 3 à 6, caractérisé en ce qu'il consiste à préparer le dérivé monohydroxylé ou monomercapto de l'oligobipyridine $BP_2$ ou $BP_3$ obtenue par le procédé selon la revendication 14 à le transformer en le sel métallique correspondant et à le faire réagir, soit avec le dérivé monohalogéné 3 ou le dimère ou trimère correspondant monohalogéné, soit avec le dérivé dihalogéné 4.

16. Procédé pour l'obtention des oligobipyridines selon la revendication 1, répondant à la formule I dans laquelle $Z_1$-Y-$Z_2$ est le groupe -CH=CH- et n est 1 ou 2, caractérisé en ce qu'il consiste à transformer le dérivé bromométhyle 5

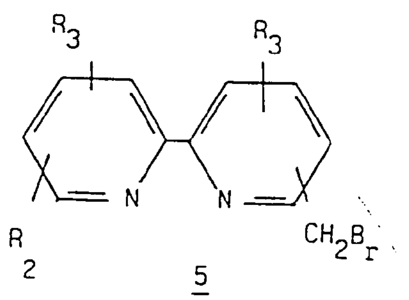

5

dans laquelle $R_2$, $R_3$ sont tels que définis dans la revendication 1 en le phosphonate 6.

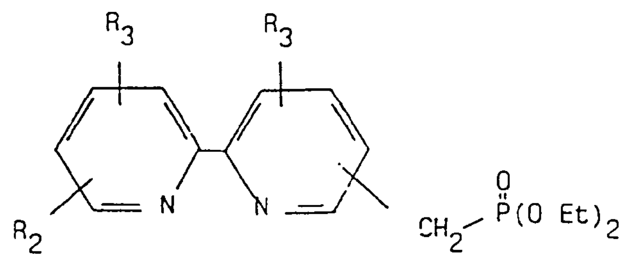

6

dans laquelle $R_2$, $R_3$ sont tels que définis dans la revendication 1, puis à faire réagir ledit phosphonate ainsi obtenu avec une bipyridine mono- ou di-aldéhyde convenablement substituée et à récupérer l'oligobipyridine ainsi formée.

17. Procédé pour l'obtention des oligobipyridines selon la revendication 1, répondant à la formule I dans laquelle $-Z_1$-Y-$Z_2$- est le groupe -CH=CH- et n est compris entre 3 et 6, caractérisé en ce qu'il consiste à préparer le dérivé monoaldéhyle de l'oligobipyridine $BP_2$ ou $BP_3$ obtenu par le procédé selon la revendication 16 et à le faire réagir avec, soit le dérivé monophosphonate 6 ou le dimère ou trimère correspondant monophosphonate, soit le diphosphonate correspondant.

18. Procédé pour l'obtention des oligobipyridines selon la revendication 1 de formule I dans laquelle $-Z_1$-Y-$Z_2$- est le groupe $-CH_2-CH_2-$, caractérisé en ce qu'il consiste à soumettre à une hydrogénation une oligobipyridine obtenue par le procédé selon l'une des revendications 16 ou 17.

## Patentansprüche

1. Neue Oligobipyridine der nachstehenden Formel I

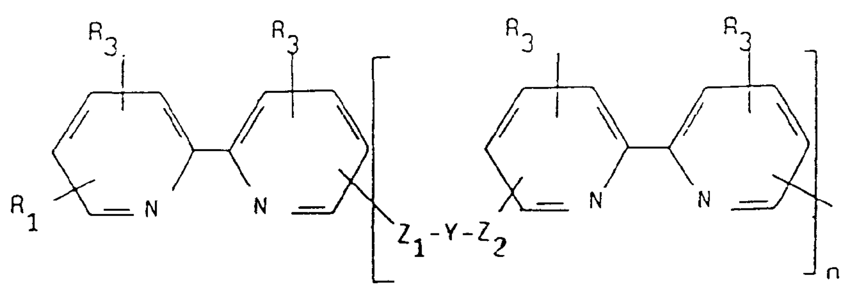

(1)

worin:

- $Z_1$ und $Z_2$, die gleich oder verschieden sind, $C_1$-$C_{12}$-Alkylengruppen darstellen;
- Y Sauerstoff oder auch Schwefel ist;
- $-Z_1$-Y-$Z_2$- die Gruppen -CH=CH- oder $-CH_2-CH_2-$ darstellt;
- n eine ganze Zahl zwischen 1 und 6 ist;
- $R_1$ und $R_2$, die gleich oder verschieden sind, $C_1$-$C_5$-Alkylgruppen, insbesondere Methyl oder Ethyl, die Reste $-Z_1YH$,
- $Z_2YH$, $-Z_1X$ oder $-Z_2X$, worin Y, $Z_1$ und $Z_2$ wie oben definiert sind und X ein Halogenatom oder eine Gruppe der Formel

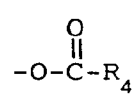

ist, worin $R_4$ eine $C_1$-$C_5$-Alkylgruppe ist, darstellen;
- $R_3$ Wasserstoff oder eine Gruppe $-COR_5$ ist, worin $R_5$ darstellt:
  . ein Halogen, vorzugsweise Chlor;
  . eine Gruppe $OR_6$, worin $R_6$ Wasserstoff oder eine $C_1$-$C_5$-Alkylgruppe bedeutet; oder

. eine Gruppe $NR_7R_8$, worin $R_7$ und $R_8$, die gleich oder verschieden sind, Wasserstoff oder eine $C_1$-$C_5$-Alkylgruppe darstellen, oder aber eines von beiden Wasserstoff bedeutet und das andere ein Nukleosid ist.

2. Verbindung nach Anspruch 1, dadurch gekennzeichnet, daß $Z_1$, $Z_2$, $R_1$, $R_2$ in der Stellung 6,6′ oder 5,5′ sind.

3. Verbindung nach einem der Ansprüche 1 oder 2, dadurch gekennzeichnet, daß die Reste $R_3$ in der Stellung 4,4′ sind.

4. Verbindung nach einem der Ansprüche 1 bis 3, dadurch gekennzeichnet, daß $Z_1$ und $Z_2$ die Gruppe $-CH_2-$ sind, Y für Sauerstoff steht und $R_1$ und $R_2$ gleich sind und Methyl bedeuten.

5. Verbindung nach einem der Ansprüche 1 bis 3, dadurch gekennzeichnet, daß $Z_1$ und $Z_2$ gleich sind und die Gruppe $-CH_2-$ darstellen, Y für Sauerstoff steht, $R_1$ Methyl ist und $R_2$ eine der nachstehenden Gruppen ist:

$-CH_2Br$, $-CH_2OH$ oder 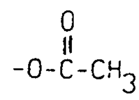

6. Verbindung nach einem der Ansprüche 1 bis 5, dadurch gekennzeichnet, daß $R_3$ Wasserstoff ist und daß n 1, 2, 3 oder 4 bedeutet.

7. Verbindung nach Anspruch 1, dadurch gekennzeichnet, daß $-Z_1-Y-Z_2-$ die Gruppe $-CH=CH-$ oder die Gruppe $-CH_2-CH_2-$ ist.

8. Verbindung nach Anspruch 7, dadurch gekennzeichnet, daß $R_3$ Wasserstoff ist, $R_1$ und $R_2$ gleich sind und Methyl darstellen.

9. Verbindung nach Anspruch 3, dadurch gekennzeichnet, daß $R_3$ die Gruppe $-COR_8$ ist, worin $R_8$ Chlor oder die Gruppe-$OR_8$ ist, wobei $R_6$ tert.Butyl oder Wasserstoff darstellt.

10. Verbindung nach Anspruch 9, dadurch gekennzeichnet, daß $R_3$ die Gruppe $-NR_7R_8$ ist, worin $R_7$ Wasserstoff bedeutet und $R_8$ ein Nukleosid ist.

11. Verbindung nach Anspruch 10, dadurch gekennzeichnet, daß $R_8$ 5′-Desoxyadenosin ist.

12. Verbindung nach Anspruch 10, dadurch gekennzeichnet, daß $R_8$ 5′-Desoxyadenosin ist.

13. Verwendung der Oligobipyridine nach einem der Ansprüche 1 bis 12 als Komplexbildner von Metallionen.

14. Verfahren zur Herstellung der Oligobipyridine nach Anspruch 1 der Formel I, worin $Z_1$ und $Z_2$ Alkylen sind, Y Sauerstoff oder Schwefel bedeutet und n 1 oder 2 ist, dadurch gekennzeichnet, daß es darin besteht, daß die Verbindung der Formel 2

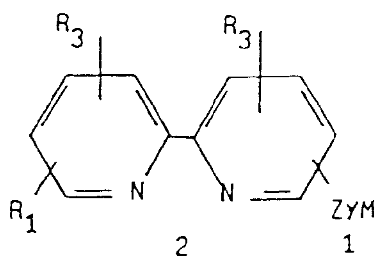

2

worin $R_1$, $R_3$, $Z_1$ und Y wie in Anspruch 1 definiert sind und M ein Metall ist, mit einer der Verbindungen der Formel 3 oder 4

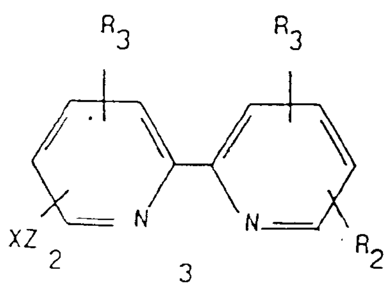

3

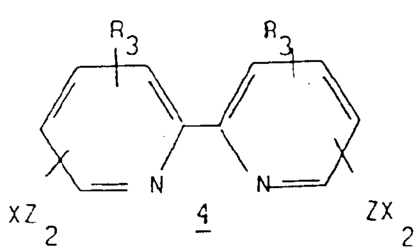

4

worin $R_2$, $R_3$, $Z_2$ und X wie in Anspruch 1 definiert sind, unter Rückfluß reagieren gelassen wird und das so

gebildete Oligobipyridin gewonnen wird.

15. Verfahren zur Herstellung der Oligobipyridine nach Anspruch 1 der Formel I, worin $Z_1$ und $Z_2$ eine Alkylengruppe sind, Y Sauerstoff oder Schwefel bedeutet und n 3 bis 6 ist, dadurch gekennzeichnet, daß es darin besteht, daß das Monohydroxyl- oder Monomercaptoderivat des durch das Verfahren nach Anspruch 14 erhaltenen Oligobipyridins $BP_2$ oder $BP_3$ hergestellt, in das entsprechende Metallsalz übergeführt und entweder mit dem Monohalogenderivat 3 oder dem entsprechenden Monohalogendimeren oder -trimeren oder mit dem Dihalogenderivat 4 reagieren gelassen wird.

16. Verfahren zur Herstellung der Oligobipyridine nach Anspruch 1 der Formel I, worin $-Z_1-Y-Z_2-$ die Gruppe -CH=CH- bedeutet und n 1 oder 2 ist, dadurch gekennzeichnet, daß es darin besteht, daß das Brommethylderivat 5

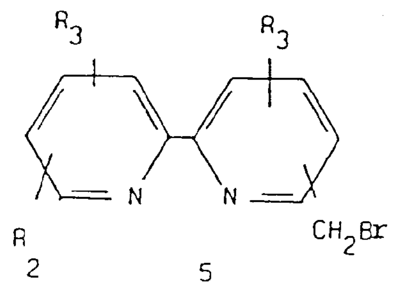

5

worin $R_2$ und $R_3$ wie in Anspruch 1 definiert sind, in das Phosphonat 6

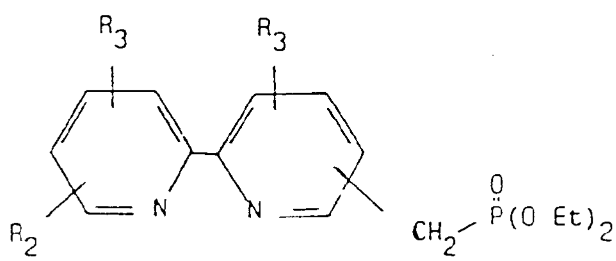

6

worin $R_2$ und $R_3$ wie in Anspruch 1 definiert sind, übergeführt wird, dann das so erhaltene Phosphonat mit einem geeignet substituierten Bipyridinmono- oder -dialdehyd reagieren gelassen wird und das so gebildete Oligobipyridin gewonnen wird.

17. Verfahren zur Herstellung der Oligobipyridine nach Anspruch 1 der Formel I, worin $-Z_1-Y-Z_2-$ die Gruppe -CH=CH- bedeutet und n 3 bis 6, dadurch gekennzeichnet, daß es darin besteht, daß das Monoaldehydderivat des durch das Verfahren nach Anspruch 16 erhaltenen Oligobipyridins $BP_2$ oder $BP_3$ hergestellt und entweder mit dem Monophosphonatderivat 6 oder dem entsprechenden Monophosphonatdimeren oder -trimeren oder mit dem entsprechenden Diphosphonat reagieren gelassen wird.

18. Verfahren zur Herstellung der Oligobipyridine nach Anspruch 1 der Formel 1, worin $-Z_1-Y-Z_2-$ die Gruppe $-CH_2-CH_2-$ ist, dadurch gekennzeichnet, daß es darin besteht, daß ein durch das Verfahren nach einem der Ansprüche 16 oder 17 erhaltenes Oligobipyridin einer Hydrierung unterzogen wird.

## Claims

1. As novel products, the oligobipyridines having the formula I below:

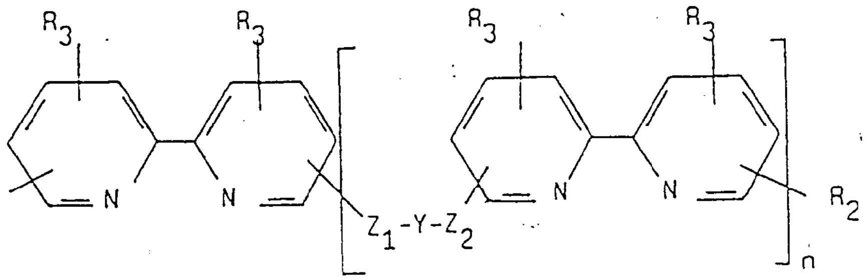

(I)

in which

– $Z_1$ and $Z_2$, which are identical or different, represent $C_1$-$C_{12}$-alkylene groups;

– Y is oxygen or sulfur; or

– $Z_1$-Y-$Z_2$- represents the groups -CH=CH- or $-CH_2-CH_2$;

– n is an integer between 1 and 6;

– $R_1$ and $R_2$, which are identical or different, represent $C_1$-$C_5$-alkyl groups, in particular the methyl group or the ethyl group, or the radicals $-Z_1YH$, $-Z_2YH$, $-Z_1X$ or

– $Z_2X$, in which Y, $Z_1$ and $Z_2$ are as defined above and X is a halogen atom or a group of the formula

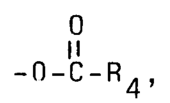

in which $R_4$ is a $C_1$-$C_5$-alkyl group; and

– $R_3$ is hydrogen or a group $-COR_5$, in which $R_5$ represents:

. a halogen, preferably chlorine;

. a group $OR_6$, in which $R_6$ represents hydrogen or a $C_1$-$C_5$-alkyl group; or

. a group $NR_7R_8$, in which $R_7$ and $R_8$, which are identical or different, represent hydrogen or a $C_1$-$C_5$-alkyl group, or one of them represents hydrogen and the other is a nucleoside.

2. Compound according to claim 1, characterized in that $Z_1$, $Z_2$, $R_1$ and $R_2$ are in the 6,6′- or 5,5′-positions.

3. Compound according to one of claims 1 or 2, characterized in that the radicals $R_3$ are in the 4,4′-positions.

4. Compound according to any one of claims 1 to 3, characterized in that $Z_1$ and $Z_2$ are the group $-CH_2-$, Y is oxygen and $R_1$ and $R_2$ are identical and represent the methyl group.

5. Compound according to any one of claims 1 to 3, characterized in that $Z_1$ and $Z_2$ are identical and represent the group $-CH_2-$, Y is oxygen, $R_1$ is the methyl group and $R_2$ is one of the following groups : $-CH_2Br$, $-CH_2OH$ or

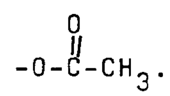

6. Compound according to any one of claims 1 to 5, characterized in that $R_3$ is hydrogen and in that n has a value of 1, 2, 3 or 4.

7. Compound according to claim 1, characterized in that $-Z_1$-Y-$Z_2$- is the group -CH=CH- or the group -CH-$CH_2$.

8. Compound according to claim 7, characterized in that $R_3$ is hydrogen and $R_1$ and $R_2$ are identical and represent the methyl group.

9. Compound according to claim 3, characterized in that $R_3$ is the group $-COR_5$ in which $R_5$ is chlorine or the group $OR_5$, $R_5$ representing the t-butyl group or hydrogen.

10. Compound according to claim 9, characterized in that $R_3$ is the group $-NR_7R_8$ in which $R_7$ is hydrogen and $R_8$ is a nucleoside.

11. Compound according to claim 10, characterized in that $R_8$ is the 5′-deoxyadenosine radical.

12. Compound according to claim 10, characterized in that $R_8$ is the 5′-deoxythymidine radical.

13. Application of the oligobipyridines according to any one of claims 1 to 12 as complexing agents for metal ions.

14. Process for the preparation of the oligobipyridines according to claim 1, having formula I in which $Z_1$

and $Z_2$ are an alkylene group, Y is oxygen or sulfur and n = 1 or 2, characterized in that it consists in reacting, under reflux, a compound of formula 2

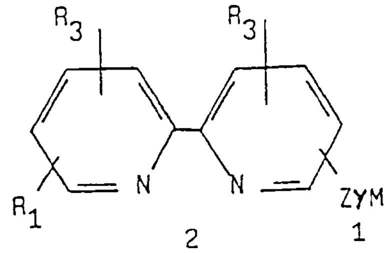

2

in which $R_1$, $R_3$, $Z_1$ and Y are as defined in claim 1 and M is a metal with one of the compounds of formulae 3 or 4

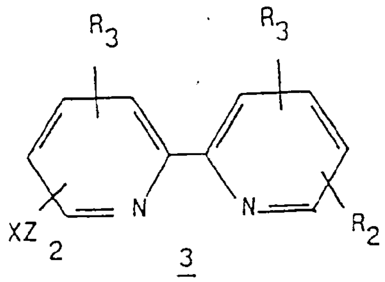

3

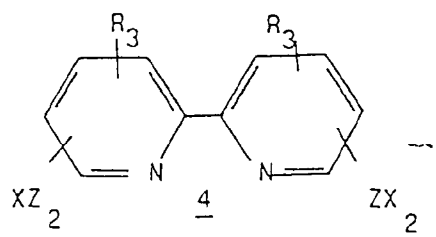

4

in which $R_2$, $R_3$, $Z_2$ and X are as defined in claim 1, and in recovering the oligobipyridine formed by this reaction.

15. Process for the preparation of the oligobipyridines according to claim 1, having formula I in which $Z_1$ and $Z_2$ are an alkylene group, Y is oxygen or sulfur and n = 3 to 6, characterized in that it consists in preparing the monohydroxylated or monomercapto derivative of the oligobipyridine $BP_2$ or $BP_3$ obtained by the process according to claim 14, in converting it into the corresponding metal salt and in reacting it either with the monohalogenated derivative 3 or the corresponding monohalogenated dimer or trimer, or with the dihalogenated derivative 4.

16. Process for the preparation of the oligobipyridines according to claim 1, having formula I in which $Z_1$-Y-$Z_2$ is the group -CH=CH- and n is 1 or 2, characterized in that it consists in converting the bromomethyl derivative 5

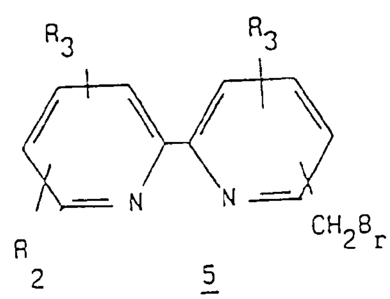

5

in which $R_2$ and $R_3$ are as defined in claim 1, into the phosphonate 6

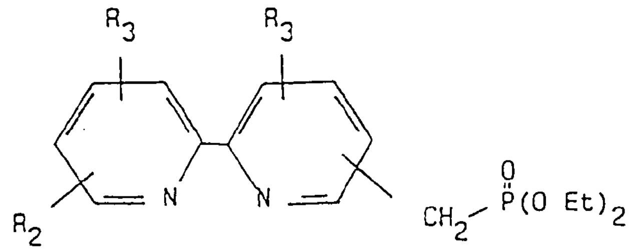

6

in which $R_2$ and $R_3$ are as defined in claim 1, in then reacting the said phosphonate obtained in this way with an appropriately substituted bipyridine monoaldehyde or dialdehyde and in recovering the oligobipyridine formed by this reaction.

17. Process for the preparation of the oligobipyridines according to claim 1, having formula I in which $-Z_1-Y-Z_2-$ is the group -CH=CH- and n is between 3 and 6, characterized in that it consists in preparing the monoaldehyde derivative of the oligobipyridine $BP_2$ or $BP_3$, obtained by the process according to claim 16, and in reacting it either with the monophosponate derivative 6 or the corresponding dimer or trimer monophosphonate, or with the corresponding diphosphonate.

18. Process for the preparation of the oligobipyridines according to claim 1, having formula I in which $-Z_1-Y-Z_2-$ is the group $-CH_2-CH_2-$, characterized in that it consists in hydrogenating an oligobipyridine obtained by the process according to one of claims 16 or 17.

Fig 1

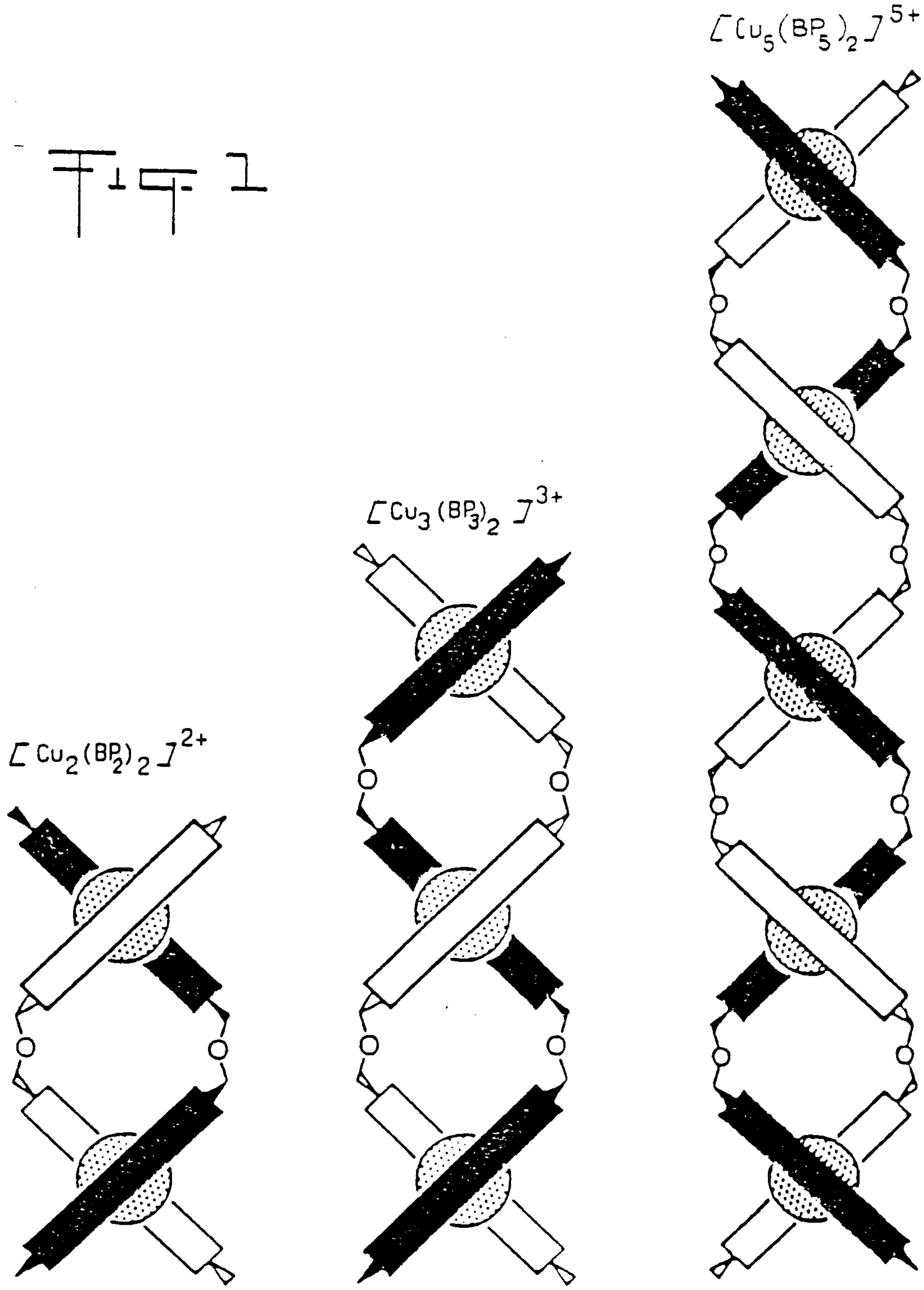

Fig. 2a

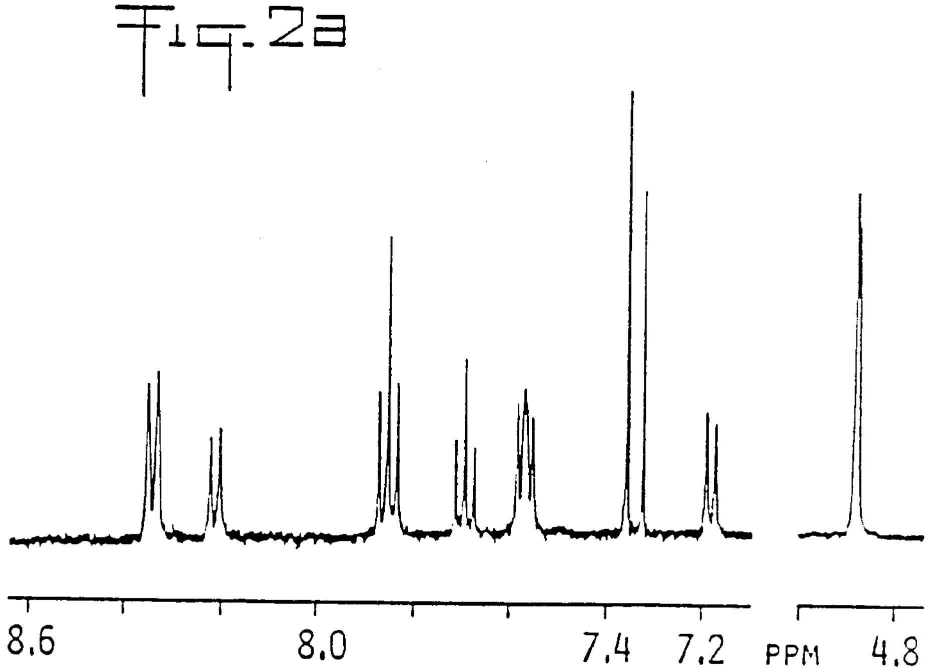


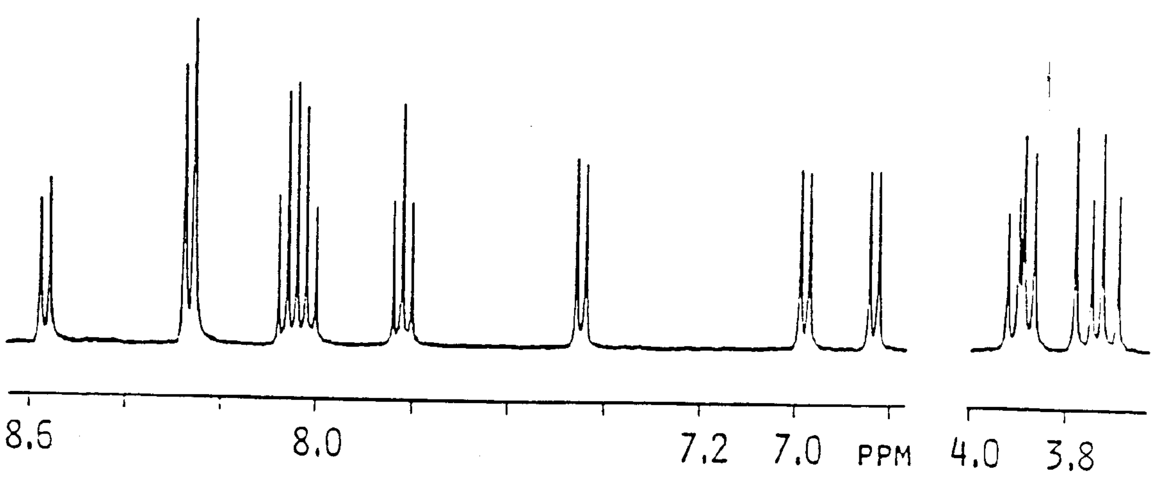


Fig. 2b

Fig. 3c

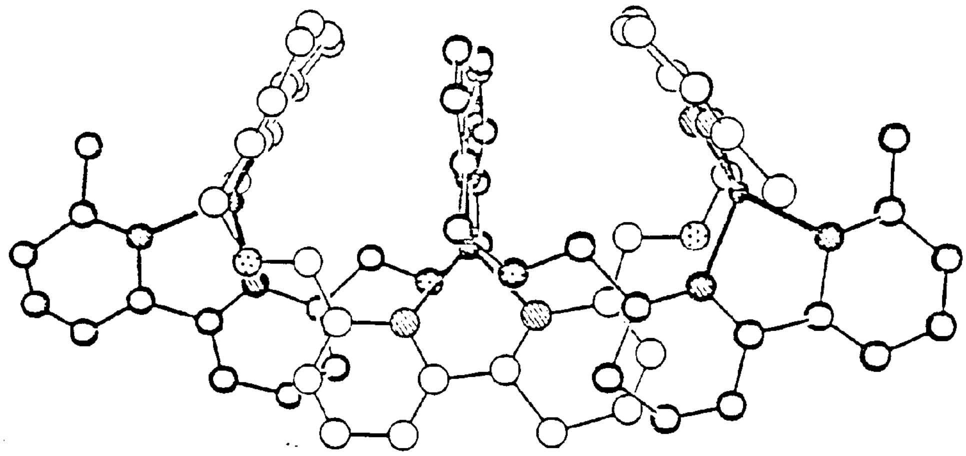

Fig. 3b

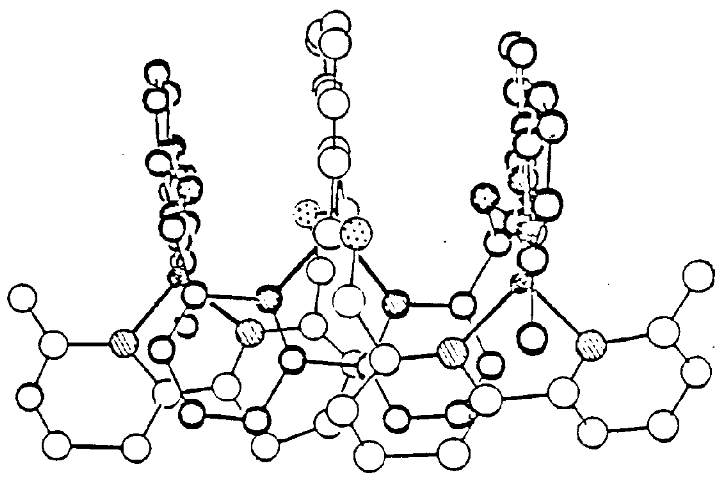

Fig. 3a

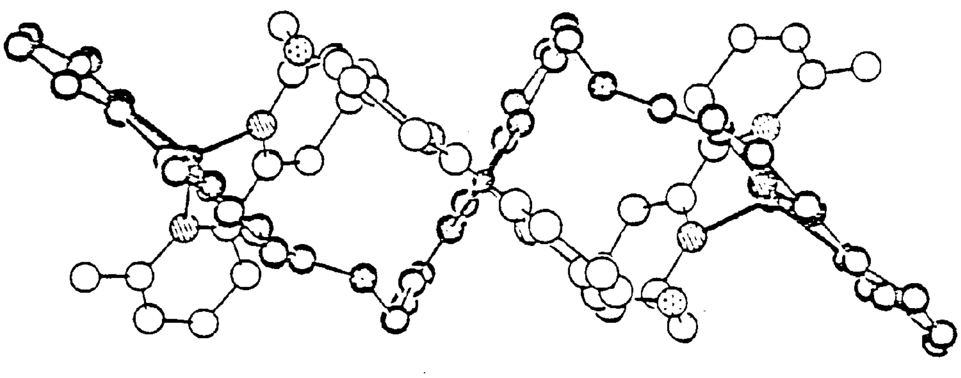

Fig. 4a

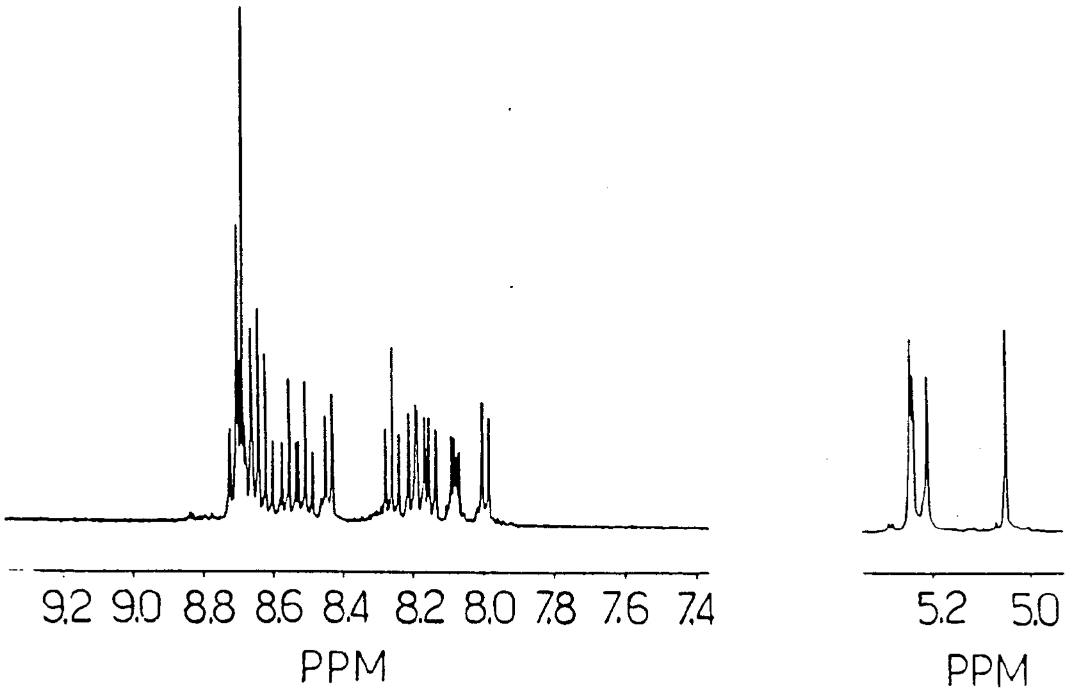
9.2 9.0 8.8 8.6 8.4 8.2 8.0 7.8 7.6 7.4
PPM
5.2 5.0
PPM

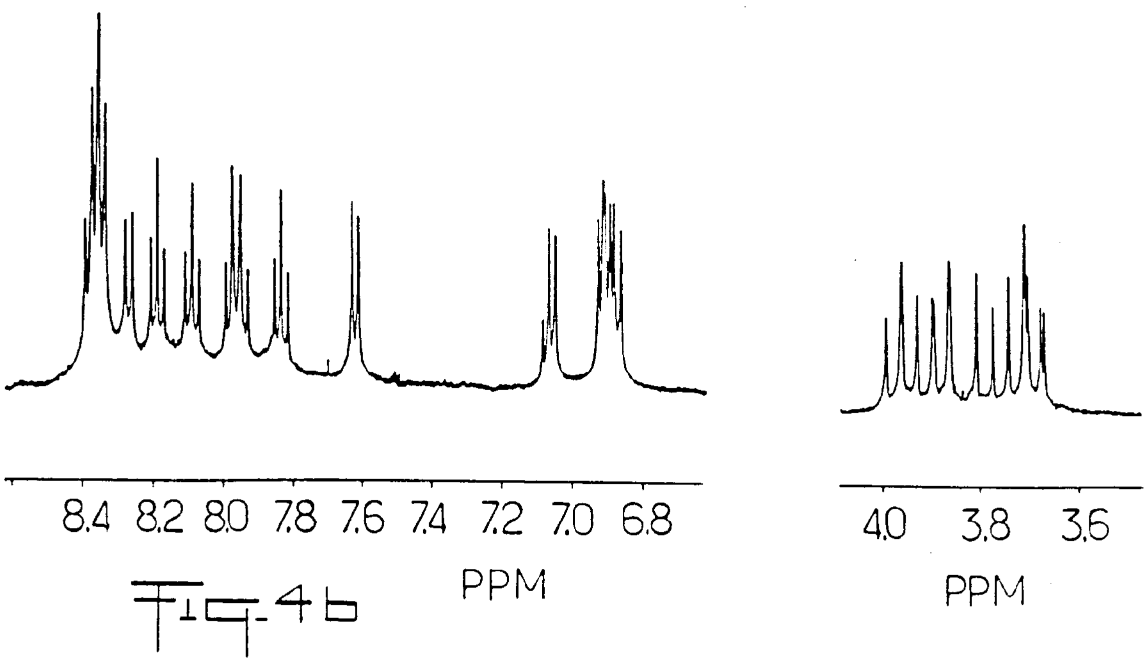
8.4 8.2 8.0 7.8 7.6 7.4 7.2 7.0 6.8
PPM
4.0 3.8 3.6
PPM

Fig. 4b